# EUROPEAN PATENT APPLICATION

(11) **EP 4 011 437 A1**
(43) Date of publication of application: **15.06.2022**
(21) Application number: 20850956.2
(22) Date of filing: 05.08.2020
(51) Int. Cl.: A61N 1/04, A61N 1/36, A41D 13/00

(54) **ELECTRICAL STIMULATION FITNESS WEAR**

(30) Priority: 06.08.2019 JP 2019144585; 13.11.2019 JP 2019205746
(71) Applicant: MTG Co., Ltd., Nagoya Aichi 453-0041 (JP)
(72) Inventor: MATSUSHITA, Tsuyoshi, Nagoya-shi Aichi 453-0041 (JP)
(74) Representative: Müller-Boré & Partner Patentanwälte PartG mbB
(86) International application number: PCT/JP2020/029934
(87) International publication number: WO 2021/025042

(57) **Abstract**

In electrical stimulation fitness wear, multiple electrode parts (30) are provided in a clothing portion, at positions that respectively face multiple target body parts such as to be respectively in proximity to the multiple target body parts, so as to provide electrical stimulation to the multiple target body parts, and the multiple electrode parts (30) each have a surface formed of a conductive polymer fabric (31). Multiple electrical cables (36) respectively connect the multiple electrode parts (30) to a connection part of a control unit electrically. The multiple electrode parts (30) include a first electrode part (30) to which a first electrical cable (36) is connected and a second electrode part (30) to which a second electrical cable 36 is connected. The first electrode part (30) and the second electrode part (30) are provided at separate positions such that a current can be applied to a certain body part between the electrodes.

## Description

### TECHNICAL FIELD

The present invention relates to clothing for exercise.

### BACKGROUND ART

Exercise devices used for human physical exercise include, for example, electrical muscle stimulation devices. The electrical muscle stimulation devices are expected to exercise and strengthen muscles by applying weak electric currents thereto to tense and relax the muscles (see Patent Literature 1, for example).

### PRIOR ART REFERENCE

### PATENT LITERTURE

Patent Literature 1: Japanese Unexamined Patent Application Publication No. 2017-6644
Patent Literature 2: Specification of Spanish Patent Application Publication No. 1129005U
Patent Literature 3: Japanese Unexamined Patent Application Publication No. 2018-7699

### SUMMARY OF INVENTION

### TECHNICAL PROBLEM

In order to develop muscles throughout the body at the same time by using multiple electrical muscle stimulation devices, fitness clothing used to fix each device to the body is known (see Patent Literature 2, for example). However, with conventional fitness clothing, not only are the electrical muscle stimulation devices and numerous electrical cables exposed from the clothing, which spoils aesthetics, but the presence of the exposed electrical cables sometimes restricts the user's movements. Meanwhile, a wearing tool including a conductive cloth portion used for a biological electrode is also known (see Patent Literature 3, for example). However, since the conductive cloth portion conducts a current within the contact surface thereof with skin, in order to ensure a sufficient amount of current flow, the area of the conductive cloth portion needs to be made large, which may restrict the shape and movement of the wear as fitness wear.

The present invention has been made in view of such a situation, and a purpose thereof is to provide fitness wear that maintains aesthetics and functionality.

### SOLUTION TO PROBLEM

In response to the above issue, electrical stimulation fitness wear according to one aspect of the present invention includes: a clothing portion wearable on a body; multiple electrode parts provided in the clothing portion, at positions that respectively face multiple target body parts such as to be respectively in proximity to the multiple target body parts, so as to provide electrical stimulation to the multiple target body parts, in which the multiple electrode parts each have a surface formed of a conductive polymer fabric; a control unit that controls a voltage of the multiple electrode parts; a connection part electrically connected to the control unit; and multiple electric wires that respectively connect the multiple electrode parts to the connection part electrically. The entirety or part of the clothing portion has a multi-layered structure in which multiple fabrics overlap each other and also includes, between the multiple fabrics, a closed space formed by sewing the entirety or part of the circumference of the closed space. The multiple electric wires include a first electric wire and a second electric wire and are arranged in the closed space. The multiple electrode parts include a first electrode part to which the first electric wire is connected and a second electrode part to which the second electric wire is connected, and the first electrode part and the second electrode part are provided at separate positions such that a current can be applied to a certain body part between the electrodes.

Another aspect of the present invention also relates to electrical stimulation fitness wear. The electrical stimulation fitness wear includes: a clothing portion that is stretchable and wearable on a body; multiple electrode parts provided at positions that respectively face multiple target body parts such as to be respectively in proximity to the multiple target body parts, so as to provide electrical stimulation to the multiple target body parts; a connection part electrically connected to a control unit that controls a voltage of the multiple electrode parts; and multiple electric wires that are stretchable and respectively connect the multiple electrode parts to the connection part electrically.

### ADVANTAGEOUS EFFECTS OF INVENTION

The present invention provides fitness wear that maintains aesthetics and functionality.

### BRIEF DESCRIPTION OF DRAWINGS

FIG. 1 is a diagram that illustrates appearance of an exercise control system;
FIG. 2 is a diagram that schematically illustrates a configuration of the exercise control system used in a first exercise program and a second exercise program;
FIG. 3 is a diagram that schematically illustrates a configuration of the exercise control system used in a third exercise program and a fourth exercise program;
FIG. 4 is a diagram that schematically illustrates appearance of fitness wear;
FIGS. 5 are diagrams that schematically illustrate arrangement of electrodes and wiring in an upper body clothing portion of the fitness wear;
FIGS. 6 are diagrams that schematically illustrate arrangement of electrodes and wiring in a lower body clothing portion of the fitness wear;
FIG. 7 is a partially enlarged view that illustrates arrangement and structures of a clothing portion, electrodes, and electrical cables;
FIG. 8 is a partially enlarged sectional view that illustrates arrangement and structures of a clothing portion, an electrode, electrical cables, a control unit, and a control unit connection part;
FIGS. 9 are external views of an electrical cable;
FIG. 10 is a partially enlarged view that illustrates a connection portion and a sewing portion of electrical cables on an inner fabric;
FIGS. 11 are explanatory sectional views that each schematically illustrate a configuration of an electrical cable;
FIGS. 12 are explanatory sectional views that each schematically illustrate an internal structure of a resin mold;
FIG. 13 is a block diagram that illustrates a functional configuration of the control unit;
FIG. 14 is a block diagram that illustrates a functional configuration of an exercise control device;
FIGS. 15 are diagrams that illustrate another example of the upper body clothing portion;
FIG. 16 is a partially enlarged sectional view that illustrates arrangement and structures of a clothing portion, an electrode, electrical cables, a control unit, and a control unit connection part in a second embodiment;
FIG. 17 is a diagram that illustrates attachment structures of a control unit and a control unit connection part in the second embodiment;
FIGS. 18 are diagrams that illustrate structures for preventing a mix-up in the second embodiment;
FIG. 19 is a diagram that illustrates structures for preventing an installation mistake in the second embodiment;
FIG. 20 is a diagram that schematically illustrates appearance of the upper body clothing portion and an upper body control unit in a first example;
FIG. 21 is a diagram that schematically illustrates arrangement of electrodes in the upper body clothing portion of the first example;
FIG. 22 is a diagram that schematically illustrates an illustrative configuration of a control unit and electrodes in the first example;
FIG. 23 is a diagram that schematically illustrates appearance of the upper body clothing portion and the upper body control unit in a second example;
FIG. 24 is a diagram that schematically illustrates an illustrative configuration of control units and electrodes in the second example;
FIG. 25 is a diagram that schematically illustrates appearance of the upper body clothing portion and the upper body control unit in a third example; and
FIG. 26 is a diagram that schematically illustrates size and arrangement of electrodes in the upper body clothing portion of a fourth example.

### DESCRIPTION OF EMBODIMENTS

### First Embodiment

Not a few users of electrical muscle stimulation devices wish to develop muscles throughout their bodies at the same time more seriously or more efficiently using the electrical muscle stimulation devices. It is also conceivable to provide an exercise program using multiple electrical muscle stimulation devices for simultaneously developing muscles throughout the body, to multiple users at the same time in a place such as a fitness gym. When multiple devices are used simultaneously for multiple body parts, it is preferable to use a simpler procedure. Also, to increase the motivation and willingness of users to exercise, pleasant aesthetics is required for each of the places where the exercise program is provided, and equipment and fitness wear to be used. However, with conventional fitness clothing, not only are the electrical muscle stimulation devices and numerous electrical cables exposed from the clothing, which spoils aesthetics, but the presence of the exposed electrical cables sometimes restricts the user's movements. When a number of electrical cables are exposed, there is also a problem that contact failure or disconnection is likely to occur in some of them. Also, if the wiring of the electrical muscle stimulation devices is exposed, wires may be hooked up when multiple users use the electrical muscle stimulation devices at the same time. In this way, such electrical components need to be installed and, in addition, putting on and taking off the clothing often requires a third party's help, and hence, handling of such conventional fitness clothing has been difficult. Further, in an environment where exercise programs are provided to a large number of users, such as a fitness gym, clothing needs to be washed repeatedly. However, conventional fitness clothing cannot be washed as it is, and the electrical components need to be detached and attached each time, which causes complication.

Therefore, the present embodiment provides fitness wear that maintains aesthetics and functionality.

Electrical stimulation fitness wear according to one aspect includes: a clothing portion wearable on a body; multiple electrode parts provided in the clothing portion, at positions that respectively face multiple target body parts such as to be respectively in proximity to the multiple target body parts, so as to provide electrical stimulation to the multiple target body parts, in which the multiple electrode parts each have a surface formed of a conductive polymer fabric; a control unit that controls a voltage of the multiple electrode parts; a connection part electrically connected to the control unit; and multiple electric wires that respectively connect the multiple electrode parts to the connection part electrically. The entirety or part of the clothing portion has a multi-layered structure in which multiple fabrics overlap each other and also includes, between the multiple fabrics, a closed space formed by sewing the entirety or part of the circumference of the closed space. The multiple electric wires include a first electric wire and a second electric wire and are arranged in the closed space. The multiple electrode parts include a first electrode part to which the first electric wire is connected and a second electrode part to which the second electric wire is connected, and the first electrode part and the second electrode part are provided at separate positions such that a current can be applied to a certain body part between the electrodes.

The "control unit" as used herein may be a removable independent control unit or may be a control device that is fixedly attached to the "connection part". The "electric wires" may be electrical cables constituted by coated conductive wires and the like and may be water-resistant. The electrode parts each have a surface formed of a conductive polymer fabric, so that they can be washed as part of the clothing portion without being removed from the clothing portion. Particularly, compared to the case of using conductive fibers containing metal such as silver, there is no concern about decreased conductivity due to oxidation or corrosion or metal allergies, for example. One of the first electrode part and the second electrode part is an anode, and the other is a cathode. The first electrode part and the second electrode part are arranged with a certain distance therebetween such as to face the same target body part. By applying a current between the electrode parts, electrical stimulation can be provided to the target body part. Especially, in the case where the area of the body part to be provided with electrical stimulation is relatively large, since the pair of a cathode and an anode are physically separated and connected to separate electric wires, even when the fabric of the clothing portion is stretched due to the body shape and movement of the user, the electrode positions can be separated or moved in the wake of the stretching. Accordingly, without providing the electrodes themselves with stretchability, interference with the stretching and contraction of the clothing portion can be prevented. Also, since the electric wires can be washed as part of the clothing portion without being removed from the clothing portion, the electric wires and the electrodes can be placed in a closed space such as to be invisible from the outside, so that the aesthetics of the fitness wear can be maintained. Furthermore, foreign matter coming into contact with the electric wires or interference with the stretching and contraction of the electric wires can be prevented.

Electrical stimulation fitness wear according to another aspect includes: a clothing portion that is stretchable and wearable on a body; multiple electrode parts provided at positions that respectively face multiple target body parts such as to be respectively in proximity to the multiple target body parts, so as to provide electrical stimulation to the multiple target body parts; a connection part electrically connected to a control unit that controls a voltage of the multiple electrode parts; and multiple electric wires that are stretchable and respectively connect the multiple electrode parts to the connection part electrically.

The "electric wires" as used herein may be stretchable electrical cables that each are configured by at least spirally winding one or more coated conductive wires formed by coating core wires with resin, around a stretchable elastic core material. The elastic core material and the coated conductive wires wound spirally therearound are stretchable in a longitudinal direction of the elastic core material, and the entire length thereof can be extended up to about 1.4 times, for example. Such electric wires can have both water resistance and stretchability, so that the electric wires can be washed as part of the clothing portion without being removed from the clothing portion. In addition, even when the fabric of the clothing portion is stretched due to the body shape and movement of the user, the electric wires can also be stretched in the wake of the stretching of the fabric, so that interference with the stretching and contraction of the clothing portion can be prevented. Also, there is no need to use long electric wires that match the stretching rate of the clothing portion, which prevents the user from having a feeling of a foreign body caused by extra electric wires in contact with the user's body when the clothing portion is contracted.

At least part of the clothing portion may have a multi-layered structure in which multiple fabrics overlap each other and may also include, between the multiple fabrics, a closed space formed by sewing at least part of the circumference thereof. The electric wires may each have stretchability such as to stretch and contract in the wake of stretching and contraction of the clothing portion, and part of the each electric wire may be fixed to a certain fixing part of at least one of the multiple fabrics. Accordingly, even when the fabric of the clothing portion is stretched due to the body shape and movement of the user, the electric wires can also be stretched in the wake of the stretching of the fabric. In addition, since part of each of the electric wires is fixed to the fixing part, even when the electric wires are pulled due to the stretching of the fabric of the clothing portion, the electric wires can be prevented from being disconnected from the electrodes and the like. Also, such structures can reduce the necessity for maintenance of the electric wires, so that the electric wires can be placed in a closed space without any problems. Therefore, the electric wires and the electrodes can be made invisible from the outside, so that the aesthetics of the fitness wear can be maintained.

The electrode parts may each include a pad member provided on the clothing portion, on the side facing the skin of the wearer, and also may include a conductive polymer fabric that covers the pad member. Each of the electric wires may be connected to a corresponding conductive polymer fabric via a connector that penetrates a fabric of the clothing portion to electrically connect the outer side and inner side thereof. The "connector" may be a metal clasp, such as a snap button, which may electrically connect the outer side and inner side of the fabric of the clothing portion such as to catch the fabric and the conductive polymer fabric. Accordingly, even when the clothing portion is configured to have a multi-layered structure in which multiple fabrics overlap each other, for example, electric wires provided within the multi-layered structure and electrodes provided on the outer side of the multi-layered structure, i.e., the side facing the skin of the wearer, can be electrically connected easily. Also, since each electrode is configured by covering the pad member with the conductive polymer fabric, protrusion of the pad member toward the skin side can prevent the electrode from separating from the skin, maintaining the contact state, while the feeling of contact at the skin can be favorably maintained. Further, since the fabric of the clothing portion has a multi-layered structure, although the inner fabric may be deformed as the convex electrodes intervene when the clothing portion is made to fit onto the user's body, deformation of the outer fabric can be restrained, so that the aesthetics of the fitness wear can be maintained.

The clothing portion may include an upper body garment and a lower body garment, in each of which multiple electrode parts and multiple electric wires are provided. To the clothing portion, a first control unit and a second control unit may be attached as the control unit. The first control unit may be attached to the front of the upper body garment, on a first side as one of the left and right sides of the midline, and the second control unit may be attached to the front of the lower body garment, on a second side that is different from the first side of the left and right sides of the midline. Since the first control unit and the second control unit are provided separately on the upper body and the lower body and also provided respectively at separate positions on the left and right sides of the midline, even when the body is bent forward or when a thigh or a knee is lifted up, the first control unit and the second control unit can be prevented from colliding and interfering with each other. Also, since the control unit is divided into the first control unit and the second control unit as independent units, the intensity of the electrical stimulation to the upper body and the intensity of the electrical stimulation to the lower body can be set separately.

In the present embodiment, users wearing electrical stimulation fitness wear are given electrical stimulation therefrom and move their bodies as instructed according to the electrical stimulation patterns, thereby realizing hybrid training that combines involuntary movement produced by electrical muscle stimulation with voluntary movement produced by consciously moving their bodies. In the hybrid training, the exercise effect can be enhanced by moving, while muscles are given electrical stimulation, the muscles in the opposite direction consciously. For example, while movement of bending an elbow is caused by electrical stimulation (involuntary movement), the exercise of consciously applying force to extend the arm is performed (voluntary movement). This can efficiently increase the muscle load in a shorter time, compared to normal exercise.

As exercise programs for hybrid training, four patterns will be mainly described. A first exercise program is performed by a single user in a store such as a fitness gym, following video instructions on an individual display or instructions provided by an instructor. A second exercise program is performed by a single user in a private place such as a home, following video instructions on a display. A third exercise program is performed by multiple users as group training in a store such as a fitness gym, simultaneously following the same video instructions or instructor's instructions. A fourth exercise program is performed by a single user in a private place such as a home synchronously with users in other places, following the same video instructions provided via a network. The second and fourth exercise programs, in particular, can realize training that is not restricted by time or environment and allows users to perform exercise programs equivalent to those in fitness gyms without visiting such specific places.

In the following, like reference characters denote like or corresponding constituting elements, members, and processes in each drawing, and repetitive description will be omitted as appropriate. Also, the dimensions of a member may be appropriately enlarged or reduced in each drawing in order to facilitate understanding. Further, in each drawing, part of members less important in describing embodiments may be omitted.

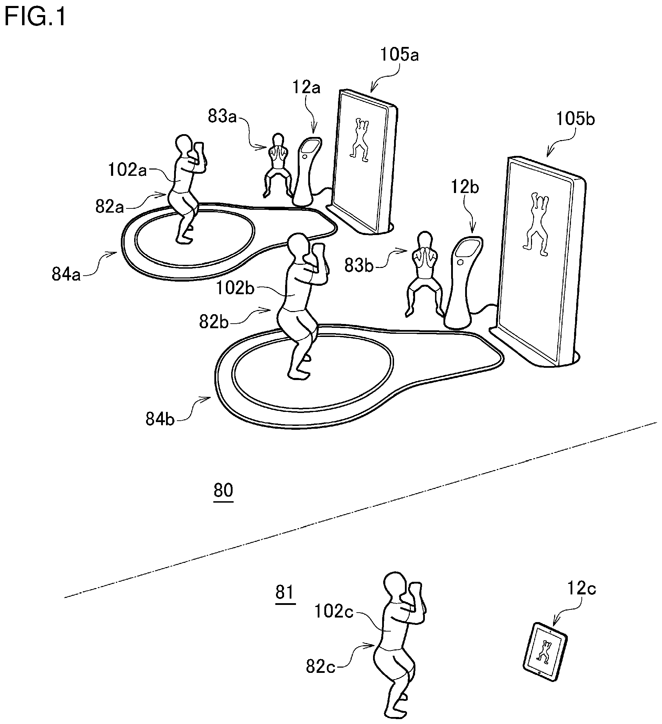

FIG. 1 illustrates appearance of an exercise control system. FIG. 1 illustrates an exercise control system mainly used in the first exercise program and the second exercise program. The exercise control system includes one or more exercise control devices 12 and one or more display devices. An exercise control device 12 used in the first exercise program is installed in a fitness gym 80 and provides an exercise program to a user 82 who is training in the fitness gym 80. FIG. 1 illustrates an example in which, in the fitness gym 80, an exercise control device 12a provides an exercise program to a user 82a, and an exercise control device 12b provides an exercise program to a user 82b. In other words, there is a one-to-one relationship between the exercise control devices 12 and users. The exercise control devices 12a and 12b are respectively connected, via wireless communication, to electrical muscle stimulation devices attached respectively to fitness wear 102a and fitness wear 102b worn by users and control the electrical muscle stimulation devices. Also, the exercise control devices 12a and 12b are connected respectively to mirror displays 105a and 105b to control the display content thereof and to display videos showing exemplary movements thereon. The exercise control devices 12a and 12b are operated by instructors 83a and 83b. The instructors 83a and 83b show exemplary movements near the exercise control devices 12a and 12b while verbally giving instructions and advice to the users 82a and 82b. The exercise control devices 12a and 12b link videos of the exemplary movements displayed on the mirror displays 105a and 105b with voltage application to the electrical muscle stimulation devices of the fitness wear 102a and fitness wear 102b, thereby achieving synchronization of voluntary movement and involuntary movement in the hybrid training.

The exercise control devices 12a and 12b may be stand-alone or may be linked to each other via a network. Also, the exercise control devices 12a and 12b may be connected to an exercise control management server via a network, such as the Internet, to provide exercise programs to the respective users based on instructions received from the exercise control management server. The exercise control devices 12a and 12b each display individual content on a corresponding mirror display 105 at individual timing, so as to individually provide exercise programs to multiple users.

Meanwhile, in the case of the exercise control system used in the second exercise program performed in a personal space such as a home, an exercise control device 12c provides an exercise program to a user 82 who is training in a personal space 81. FIG. 1 further illustrates an example of providing an exercise program also to a user 82c in the personal space 81. The exercise control device 12c used in the personal space 81 is connected, via wireless communication, to an electrical muscle stimulation device attached to fitness wear 102c and controls the electrical muscle stimulation device. The exercise control device 12c is an electronic device, such as a tablet terminal, that also serves as a display.

The exercise control devices 12a-12c may be connected to an exercise control management server via a network, such as the Internet, to provide exercise programs to the respective users 82 based on instructions received from the exercise control management server. The exercise control devices 12a-12c each display individual content on a corresponding mirror display 105 or the exercise control device 12c at individual timing, so as to individually provide exercise programs to multiple users. Each of the exercise control devices 12a-12c may be configured such that, without connection with the exercise control device 12 via wireless communication, an exercise program of a corresponding electrical muscle stimulation device can be started or finished by using buttons provided on the electrical muscle stimulation device or by operating a remote controller, for example, and an exercise program can be executed independently without instructions from the exercise control device 12.

In the third exercise program, multiple users are provided with the same exercise program simultaneously by a single exercise control device 12 and a single display device 106. In the exercise control system used in the fourth exercise program, the exercise control device 12c connected via a network is further connected to the exercise control device 12 and provided with the same exercise program in synchronization with the exercise control device 12.

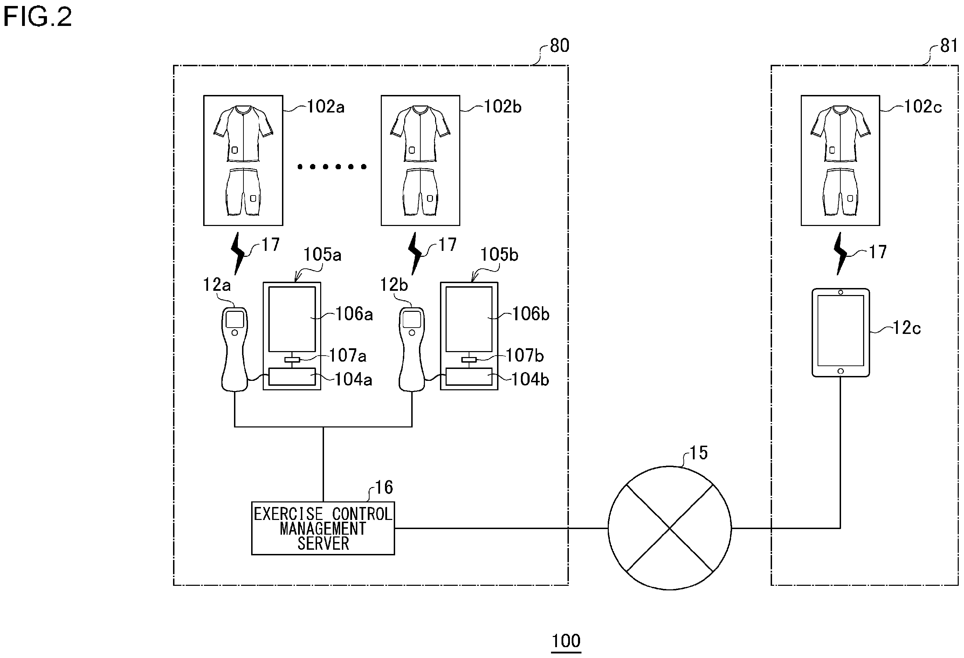

FIG. 2 schematically illustrates a configuration of the exercise control system used in the first exercise program and the second exercise program. An exercise control system 100 includes multiple sets of fitness wear 102, multiple exercise control devices 12, multiple mirror displays 105, and an exercise control management server 16. In fitness wear 102, multiple electrodes as an electrical muscle stimulation device are built, and a control unit is attached to the fitness wear 102. In FIG. 2, the fitness wear 102a and fitness wear 102b, the exercise control devices 12a and 12b, the mirror displays 105a and 105b, and the exercise control management server 16 are installed or used in the fitness gym 80, among which the fitness wear 102a and fitness wear 102b are lent to users. In contrast, the fitness wear 102c and the exercise control device 12c (the terminal also serves as a display) are used in the personal space 81. The fitness wear 102c and the exercise control device 12c are different from the fitness wear 102a and fitness wear 102b, the exercise control devices 12a and 12b, and the mirror displays 105a and 105b used in the fitness gym 80 in that they are owned and used by the user him or herself.

In the fitness gym 80, the fitness wear 102a and the exercise control device 12a are connected, and the fitness wear 102b and the exercise control device 12b are connected, via short-range wireless communication 17 such as Bluetooth (registered trademark). Also, the exercise control devices 12a and 12b are connected respectively to the mirror displays 105a and 105b by wired cables. For example, when each of the mirror displays 105 is configured to have a monitor and a personal computer built therein, the exercise control devices 12a and 12b are connected to the respective personal computers by wired cables, such as LAN cables and USB cables. When each of the mirror displays 105 is constituted substantially by a monitor only without having a personal computer built therein, the exercise control devices 12a and 12b are connected to the respective monitors by wired cables, such as monitor cables. The exercise control devices 12a and 12b are connected to the exercise control management server 16 via wired or wireless communication. As a modification, each exercise control device 12 may operate in a stand-alone manner or may be connected to the exercise control management server 16 without being linked thereto and operate individually. In the example of FIG. 2, a single exercise control management server 16 installed in the fitness gym 80 supervises all the exercise control devices 12 in the fitness gym 80. The exercise control management server 16 may also control the operations of all the exercise control devices 12 and the electrical muscle stimulation devices. Also, the exercise control management server 16 may be provided outside the fitness gym 80, such as on the Internet.

In the personal space 81, the fitness wear 102c and the exercise control device 12c are connected via the short-range wireless communication 17 such as Bluetooth (registered trademark). The exercise control device 12c is exemplified by a tablet terminal that also serves as a display but may be an information terminal, such as a cellular phone, or a personal computer connected to a monitor. As a modification of the exercise control device 12c, a dedicated small terminal with functions equivalent to those of the exercise control devices 12a and 12b may be employed. The exercise control device 12c is connected to a network 15 via wireless or wired communication and also connected to the exercise control management server 16 via the network 15. Accordingly, an exercise program carried out in the fitness gym 80 can also be carried out in the personal space 81.

An exercise control device 12 and a mirror display 105 are regarded as a set, and one or more sets are installed in the fitness gym 80. One set is used by one user, and a set of fitness wear 102, a top and a bottom, is also provided for the user. The fitness wear 102 includes an upper body clothing portion and a lower body clothing portion. In the fitness wear 102, electrodes of an electrical muscle stimulation device are provided such as to provide electrical stimulation to body parts including, for example, abdominal muscles, flanks, arms, legs, and buttocks of the user, on the back side of the corresponding areas (i.e., the side that can come in contact with the user's skin). A control unit of the electrical muscle stimulation device is attached to each of the upper body clothing portion and the lower body clothing portion of the fitness wear 102 to provide electrical stimulation to the user's muscles. The electrical muscle stimulation device includes multiple electrodes and cables, and a control unit that communicates with an exercise control device 12 via short-range wireless communication such as Bluetooth (registered trademark) to send and receive information. The control unit is attached to each of the upper body clothing portion and the lower body clothing portion, but, as a modification, a single control unit for the entire fitness wear 102, a set of a top and a bottom, may be attached to either the upper body clothing portion or the lower body clothing portion to control the application of electrical stimulation to the electrodes throughout the body. An exercise control program, of which the start of execution is ordered through communication with the exercise control device 12, controls the voltage setting and operation in the electrical muscle stimulation device. According to an instruction of the exercise control program executed by the exercise control device 12, a display control device 104 displays an exercise video on the display device 106.

Each of the exercise control devices 12a and 12b is an information terminal operated by an instructor or a user in the fitness gym 80. The exercise control devices 12a and 12b control the intensity setting and operation for electrical stimulation applied by the electrical muscle stimulation devices provided in the fitness wear 102a and fitness wear 102b, via the control units for the respective sets of fitness wear. The exercise control device 12c is an information terminal operated by a user in the personal space 81 and controls, via a control unit, the intensity setting and operation for electrical stimulation applied by the electrical muscle stimulation device provided in the fitness wear 102c.

Each mirror display 105 includes the display control device 104, the display device 106, and a posture sensor 107. The exercise control devices 12a and 12b display explanation of exercise programs and videos of exemplary movements on the display devices 106a and 106b via control by the display control devices 104a and 104b. However, considering the delay of wireless communication with each electrical muscle stimulation device, for example, instead of displaying a movement video at timing completely synchronized with a control pulse of the electrical muscle stimulation device, the video may be displayed at timing delayed by a predetermined period. The posture sensors 107a and 107b each include a camera capable of capturing user's movements and capture a video of a user performing exercise according to an exercise program. The display control devices 104a and 104b display, on the display devices 106a and 106b, such a video or a video in which skeletal movement is reproduced based on the analysis of the user's movements.

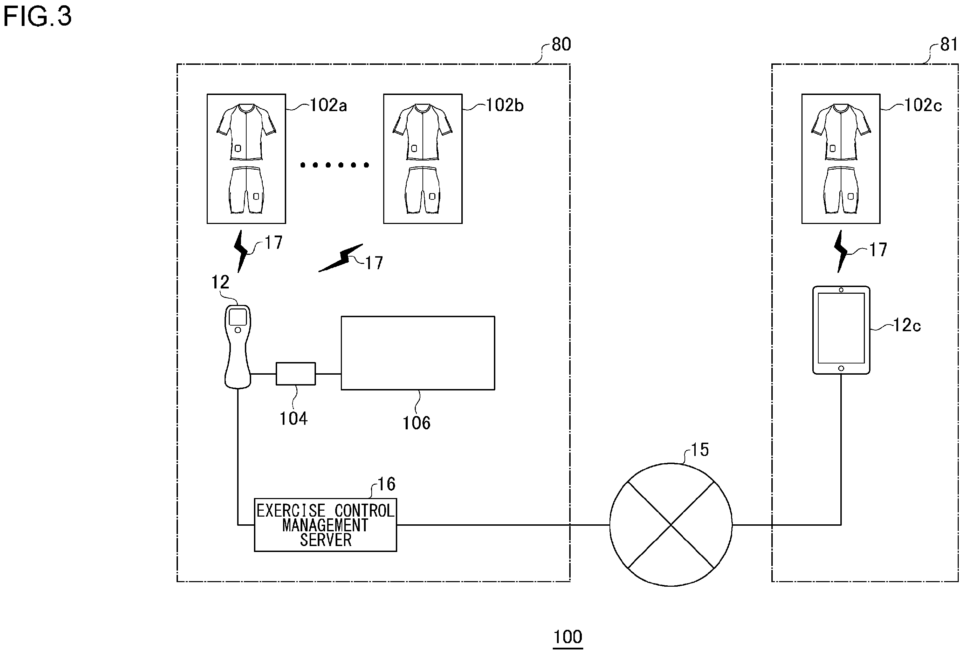

FIG. 3 schematically illustrates a configuration of the exercise control system used in the third exercise program and the fourth exercise program. As described previously, in the third exercise program, the same exercise program is provided simultaneously to multiple users in the fitness gym 80; in the fourth exercise program, the same exercise program is also provided synchronously to a user in the personal space 81 via a network. The exercise control system 100 used in the third exercise program includes multiple sets of fitness wear 102a and fitness wear 102b to be worn by multiple users in the fitness gym 80, a single exercise control device 12 connected to the multiple sets of fitness wear 102 by communication, a display device 106, a display control device 104, and the exercise control management server 16. The exercise control system 100 used in the fourth exercise program further includes fitness wear 102c and an exercise control device 12c used in the personal space 81.

The exercise control device 12 used in the fitness gym 80 functions as a hub for multiple electrical muscle stimulation devices for multiple users. Accordingly, it is more efficient for an instructor or users to operate the exercise control device 12 to collectively control the multiple electrical muscle stimulation devices than to individually set up and operate the multiple electrical muscle stimulation devices. In addition, movements for multiple users can be synchronized and simultaneously controlled with the same program. The exercise control device 12 displays, on the display device 106 via the display control devices 104, explanation of an exercise program and a video of exemplary movements synchronized with the control of the multiple electrical muscle stimulation devices. Alternatively, the same content may be displayed on multiple display devices 106 synchronously so as to provide an exercise program simultaneously in parallel. The exercise control device 12c is connected to the network 15 via wireless or wired communication and also connected to the exercise control management server 16 via the network 15. The exercise control device 12c may synchronously display the same content thereon so as to provide the exercise program simultaneously in parallel with the exercise control device 12 in the fitness gym 80. Accordingly, an exercise program provided to multiple users and carried out in the fitness gym 80 can also be carried out in the personal space 81 almost simultaneously. However, providing the exercise program to the user 82c in the personal space 81 may only be optional, and the exercise program can be carried out only by providing it to the users 82a and 82b in the fitness gym 80.

Multiple exercise control management servers 16 may be installed and may be connected to each other via wired or wireless communication such that the operations of multiple exercise control devices 12 and multiple electrical muscle stimulation devices supervised by the exercise control management servers 16 are linked. The multiple exercise control management servers 16 may be connected and linked to another exercise control management server installed in another fitness gym 80 via the network 15 or may be connected to a certain management server on the network 15 that supervises the multiple exercise control management servers.

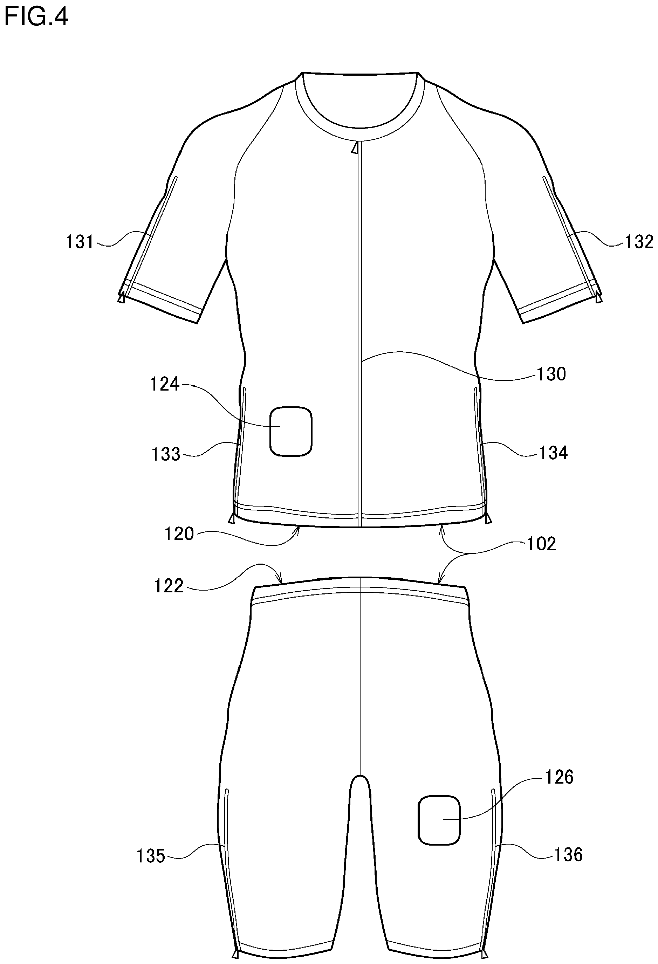

FIG. 4 schematically illustrates appearance of the fitness wear. The fitness wear 102 is constituted by a combination of an upper body clothing portion 120 that is wearable on a user's upper body and a lower body clothing portion 122 that is wearable on a user's lower body. The upper body clothing portion 120 is an open-front short-sleeved jacket of which the front is opened and closed with a front fastener 130 provided along the midline. The upper body clothing portion 120 and the lower body clothing portion 122 are garments made of chemical fiber fabric, which is shaped to be wearable on the body and has electrical insulation, and have high stretchability required to fit onto the body regardless of the body shape of the wearer. The upper body clothing portion 120 and the lower body clothing portion 122 are worn directly on the bare skin without any inner wear. On the inner side of the upper body clothing portion 120 and the lower body clothing portion 122, multiple electrodes are provided. The arrangement of the electrodes will be described later. On the outside of the right sleeve, a right arm fastener 131 is provided from the cuff toward the tip of the shoulder, and a left arm fastener 132 is similarly provided on the outside of the left sleeve from the cuff toward the tip of the shoulder. By closing the right arm fastener 131 and the left arm fastener 132, sleeve areas can be made to fit more closely onto the arms. On the inner side of each of the right and left cuffs, an anti-slip rubber material is attached along the inner circumference of the cuff, preventing the cuff from rolling or being rolled up during exercise. On the right flank, a right flank fastener 133 is provided from the hem toward the armpit, and a left flank fastener 134 is similarly provided on the left flank from the hem toward the armpit. By closing the right flank fastener 133 and the left flank fastener 134, hem areas can be made to fit more closely around the torso. Also on the inner side of the hem, an anti-slip rubber material is attached along the inner circumference of the hem, preventing the hem from rolling or being rolled up during exercise. Further, on the outside of the right leg, a right leg fastener 135 is provided from the hem toward the right hip, and a left leg fastener 136 is similarly provided on the outside of the left leg from the hem toward the left hip. By closing the right leg fastener 135 and the left leg fastener 136, hem areas can be made to fit more closely onto the legs. Also on the inner side of each of the right and left hems, an anti-slip rubber material is attached along the inner circumference of the hem, preventing the hem from rolling or being rolled up during exercise. Thus, with the fasteners provided at the respective positions of the upper body clothing portion 120 and the lower body clothing portion 122, tightening of the fasteners and stretchability of the fabric allows the entire wear to fit onto the body evenly, so that the contact states of the electrodes with the skin can be favorably maintained. Also, by adjusting the degree of closing (or opening) of each fastener, the degree of fit onto the body can be adjusted to suit the user's preference and body shape.

At a position in front of the right flank on the upper body clothing portion 120, an upper body control unit 124 is attached. Similarly, at a position on the front side of the left leg on the lower body clothing portion 122, a lower body control unit 126 is attached. The upper body control unit 124 and the lower body control unit 126 control a voltage applied to each electrode. The upper body control unit 124 and the lower body control unit 126 are installed as independent units provided separately for the upper body and the lower body and exposed to the outside. By separating the control units for the upper body and the lower body, the upper body and the lower body can be trained at different exercise intensities (set voltage values), and some users may selectively purchase or use only one of the electrical muscle stimulation device for the upper body and that for the lower body. Also, the upper body control unit 124 and the lower body control unit 126 can be linked by pairing them via short-range wireless communication when they are activated. In this case, when one of the upper body control unit 124 and the lower body control unit 126 is operated, the other also operates therewith, so that the electrical muscle stimulation devices for the upper body and the lower body can be collectively controlled. By pairing the upper body control unit 124 and the lower body control unit 126 with an information terminal (such as a cellular phone and a tablet terminal) as an exercise control device 12 via short-range wireless communication when they are activated, the upper body control unit 124 and the lower body control unit 126 can also be controlled by means of a control application installed on the information terminal. In this case, the upper body control unit 124 and the lower body control unit 126 can be operated separately or can also be operated collectively. The upper body control unit 124 and the lower body control unit 126 are attached respectively at positions on the right side and the left side of the midline and are hence positioned diagonally to each other on the front of the body. Therefore, even when the body is bent forward or when a thigh or a knee is lifted up, the upper body control unit 124 and the lower body control unit 126 can be prevented from colliding and interfering with each other.

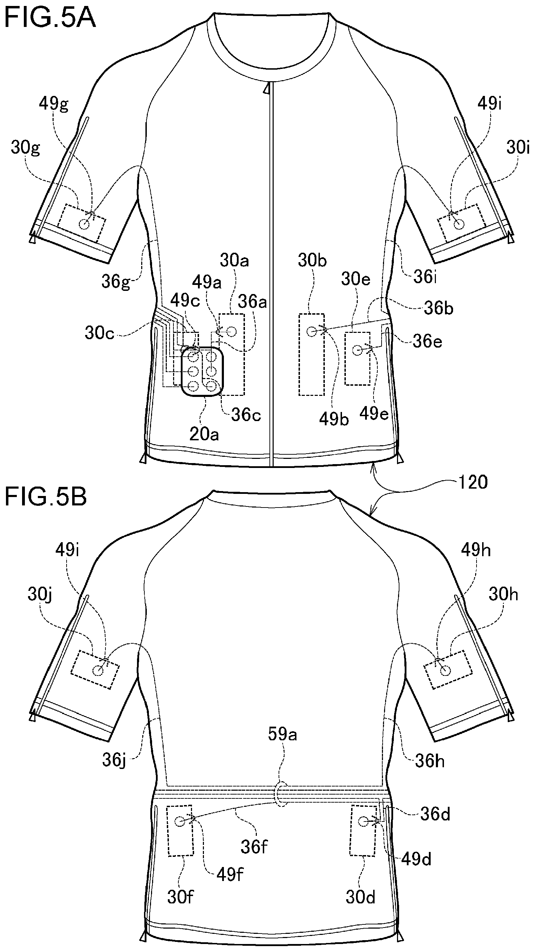

FIGS. 5 schematically illustrate arrangement of electrodes and wiring in the upper body clothing portion of the fitness wear. FIG. 5A illustrates the front side of the upper body clothing portion 120, and FIG. 5B illustrates the back side of the upper body clothing portion 120. The electrodes are arranged on the inner side of the fabric of the upper body clothing portion 120, and at least part of the fabric has a multi-layered structure in which the outer fabric and the inner fabric overlap each other and electrical cables are provided therebetween. The electrical cables are water-resistant and stretchable. Since the arrangement and wiring of the electrodes, electrical cables, and the like is not actually visible from the outside, they are illustrated with dotted lines for explanation.

There are multiple electrode regions on the inner side of the upper body clothing portion 120, in areas corresponding to target body parts to be electrically stimulated, such as areas corresponding to the user's abdominal muscles, flanks, arms, and other body parts. Electrodes are provided at separate positions such that a current can be applied to specific body parts between the electrodes. Each of the multiple electrodes is electrically connected to a first control unit connection part 20a by an individual electrical cable 36. The first control unit connection part 20a is provided at a position corresponding to the upper body control unit 124 shown in FIG. 4, and, when the upper body control unit 124 is attached to the first control unit connection part 20a, the first control unit connection part 20a and the upper body control unit 124 are electrically connected. Each of the multiple electrical cables 36 connected to an electrode is stretchable and twisted with a cable fixing tape 49, which is sewn onto the upper body clothing portion 120 at a position preceding the electrode, to be fixed in position; accordingly, even when the electrical cable 36 is pulled in the wake of stretching or contraction of the upper body clothing portion 120, the electrical cable 36 can be prevented from being disconnected from the electrode.

In an area corresponding to the abdominal muscles, as a pair of a cathode and an anode arranged laterally such that the rectus abdominis muscles are positioned therebetween, a first electrode part 30a is provided on the right, and a second electrode part 30b is provided on the left. When a voltage is applied between the electrodes via a first electrical cable 36a and a second electrical cable 36b by the upper body control unit 124, electrical stimulation is provided to the rectus abdominis muscles. At a position preceding the first electrode part 30a, a first cable fixing tape 49a is sewn, and the first electrical cable 36a, which connects the first electrode part 30a and the first control unit connection part 20a, is twisted with the first cable fixing tape 49a such as to be fixed in position. Also, at a position preceding the second electrode part 30b, a second cable fixing tape 49b is sewn, and the second electrical cable 36b, which is provided from the second electrode part 30b on the left abdominal muscle to the first control unit connection part 20a on the front side through the left flank, the back, and the right flank, is twisted with the second cable fixing tape 49b such as to be fixed in position.

In an area corresponding to the right flank, as a pair of a cathode and an anode arranged along a back and forth direction such that the right oblique abdominal muscle is positioned therebetween, a third electrode part 30c is provided on the front side, and a fourth electrode part 30d is provided on the back side. When a voltage is applied between the electrodes via a third electrical cable 36c and a fourth electrical cable 36d by the upper body control unit 124, electrical stimulation is provided to the right oblique abdominal muscle. At a position preceding the third electrode part 30c, a third cable fixing tape 49c is sewn, and the third electrical cable 36c, which connects the third electrode part 30c and the first control unit connection part 20a, is twisted with the third cable fixing tape 49c such as to be fixed in position. Also, at a position preceding the fourth electrode part 30d, a fourth cable fixing tape 49d is sewn, and the fourth electrical cable 36d, which is provided from the fourth electrode part 30d on the back side to the first control unit connection part 20a on the front side through the right flank, is twisted with the fourth cable fixing tape 49d such as to be fixed in position.

In an area corresponding to the left flank, as a pair of a cathode and an anode arranged along a back and forth direction such that the left oblique abdominal muscle is positioned therebetween, a fifth electrode part 30e is provided on the front side, and a sixth electrode part 30f is provided on the back side. When a voltage is applied between the electrodes via a fifth electrical cable 36e and a sixth electrical cable 36f by the upper body control unit 124, electrical stimulation is provided to the left oblique abdominal muscle. The fifth electrical cable 36e is provided from the fifth electrode part 30e on the left flank on the front side through the back to be connected to the fourth electrode part 30d, so as to be electrically connected to the first control unit connection part 20a via the fourth electrode part 30d. At a position preceding the fifth electrode part 30e, a fifth cable fixing tape 49e is sewn, and the fifth electrical cable 36e is twisted with the fifth cable fixing tape 49e such as to be fixed in position. Further, the fifth electrical cable 36e is also twisted with the fourth cable fixing tape 49d sewn at a position preceding the fourth electrode part 30d such as to be fixed in position. Also, at a position preceding the sixth electrode part 30f, a sixth cable fixing tape 49f is sewn, and the sixth electrical cable 36f, which is provided from the sixth electrode part 30f on the left flank on the back side to the first control unit connection part 20a on the front side through the back and the right flank, is twisted with the sixth cable fixing tape 49f such as to be fixed in position.

In an area corresponding to the right arm, as a pair of a cathode and an anode arranged vertically such that the right biceps and triceps brachii muscles are sandwiched therebetween, a seventh electrode part 30g is provided on the upper side (front side), and an eighth electrode part 30h is provided on the lower side (back side). When a voltage is applied between the electrodes via a seventh electrical cable 36g and an eighth electrical cable 36h by the upper body control unit 124, electrical stimulation is provided to the right biceps and triceps brachii muscles. The seventh electrode part 30g and the eighth electrode part 30h vertically sandwich the biceps and triceps brachii muscles, but the size of each electrode is not made larger than necessary. The seventh electrode part 30g is disposed at a position shifted anteriorly (toward the elbow) from the most prominent part of the biceps brachii muscle, and the eighth electrode part 30h is disposed at a position shifted posteriorly (toward the shoulder) from the most prominent part of the triceps brachii muscle. This allows the biceps and triceps brachii muscles to be sandwiched not only vertically but also in the back and forth directions, increasing the stability of the placement and also preventing both the electrodes shifting simultaneously from the prominent parts of the muscles toward the shoulder due to vibration of the electrical stimulation. Also, since the biceps and triceps brachii muscles are electrically stimulated along a diagonal line extending upward or downward and also forward or backward, each muscle can be efficiently provided with a load using electrodes with relatively small areas and relatively little electricity. Furthermore, the biceps and triceps brachii muscles can be electrically stimulated using a small number of electrodes and wires, reducing interference with arm movement. At a position preceding the seventh electrode part 30g, a seventh cable fixing tape 49g is sewn, and the seventh electrical cable 36g, which is provided from the seventh electrode part 30g to the first control unit connection part 20a through the right armpit on the front side, is twisted with the seventh cable fixing tape 49g such as to be fixed in position. Also, at a position preceding the eighth electrode part 30h, an eighth cable fixing tape 49h is sewn, and the eighth electrical cable 36h, which is provided from the eighth electrode part 30h to the first control unit connection part 20a through the right armpit on the back side, is twisted with the eighth cable fixing tape 49h such as to be fixed in position.

In an area corresponding to the left arm, as a pair of a cathode and an anode arranged vertically such that the left biceps and triceps brachii muscles are sandwiched therebetween, a ninth electrode part 30i is provided on the upper side (front side), and a tenth electrode part 30j is provided on the lower side (back side). When a voltage is applied between the electrodes via a ninth electrical cable 36i and a tenth electrical cable 36j by the upper body control unit 124, electrical stimulation is provided to the left biceps and triceps brachii muscles. The ninth electrode part 30i and the tenth electrode part 30j vertically sandwich the biceps and triceps brachii muscles, but the size of each electrode is not made larger than necessary. The ninth electrode part 30i is disposed at a position shifted anteriorly (toward the elbow) from the most prominent part of the biceps brachii muscle, and the tenth electrode part 30j is disposed at a position shifted posteriorly (toward the shoulder) from the most prominent part of the triceps brachii muscle. This allows the biceps and triceps brachii muscles to be sandwiched not only vertically but also in the back and forth directions, increasing the stability of the placement and also preventing both the electrodes shifting simultaneously from the prominent parts of the muscles toward the shoulder due to vibration of the electrical stimulation. Also, since the biceps and triceps brachii muscles are electrically stimulated along a diagonal line extending upward or downward and also forward or backward, each muscle can be efficiently provided with a load using electrodes with relatively small areas and relatively little electricity. Furthermore, the biceps and triceps brachii muscles can be electrically stimulated using a small number of electrodes and wires, reducing interference with arm movement. At a position preceding the ninth electrode part 30i, a ninth cable fixing tape 49i is sewn, and the ninth electrical cable 36i, which is provided from the ninth electrode part 30i to the first control unit connection part 20a through the left armpit on the front side, the left flank, the back, and the right flank, is twisted with the ninth cable fixing tape 49i such as to be fixed in position. Also, at a position preceding the tenth electrode part 30j, a tenth cable fixing tape 49j is sewn, and the tenth electrical cable 36j, which is provided from the tenth electrode part 30j to the first control unit connection part 20a through the left armpit on the back side, the left flank, the back, and the right flank, is twisted with the tenth cable fixing tape 49j such as to be fixed in position.

The five electrical cables 36 of the tenth electrical cable 36j connecting the tenth electrode part 30j and the first control unit connection part 20a, the ninth electrical cable 36i connecting the ninth electrode part 30i and the first control unit connection part 20a, the second electrical cable 36b connecting the second electrode part 30b and the first control unit connection part 20a, the fourth electrical cable 36d connecting the fifth electrode part 30e and the fourth electrode part 30d, and the sixth electrical cable 36f connecting the sixth electrode part 30f and the first control unit connection part 20a are provided from the back side to the front side through the vicinity of the center of the back and through the right flank. Near the center of the back, a cable fixing tape 59a for bundling the five electrical cables 36 is disposed, so that the five electrical cables 36 are bundled by the cable fixing tape 59a near the center of the back. The cable fixing tape 59a may be sewn and fixed near the center of the back of the upper body clothing portion 120.

With the arrangement and wiring as described above, the electrical cables can be provided without exposure to the outside. Also, since the configurations other than the upper body control unit 124 are water-resistant, only by removing the upper body control unit 124, the fitness wear 102 can be washed without removing the electrode parts 30 and the electrical cables 36. Although FIGS. 5 illustrate an example of the upper body clothing portion 120 in which the electrodes are arranged mainly for men, the arrangement and the size of the electrodes, for example, may be different for women.

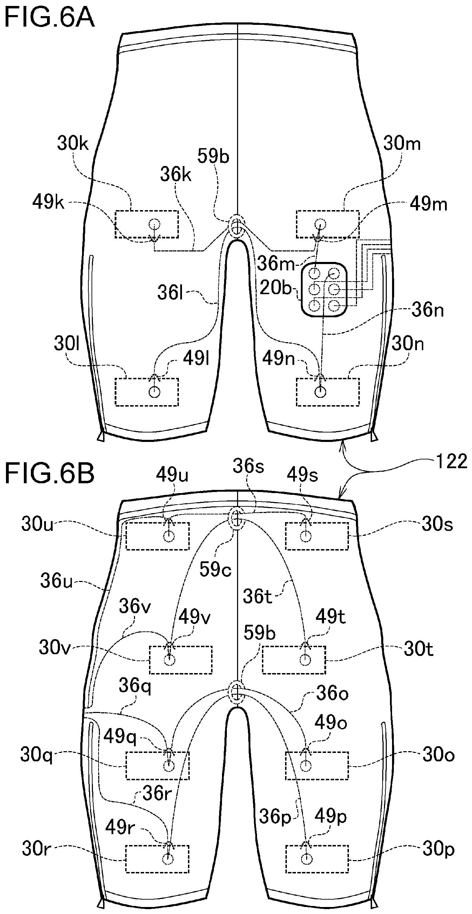

FIGS. 6 schematically illustrate arrangement of electrodes and wiring in the lower body clothing portion of the fitness wear. FIG. 6A illustrates the front side of the lower body clothing portion 122, and FIG. 6B illustrates the back side of the lower body clothing portion 122. The electrodes are arranged on the inner side of the fabric of the lower body clothing portion 122, and at least part of the fabric has a multi-layered structure in which the outer fabric and the inner fabric overlap each other and electrical cables are provided therebetween. The electrical cables are water-resistant and stretchable. Since the arrangement and wiring of the electrodes, electrical cables, and the like is not actually visible from the outside, they are illustrated with dotted lines for explanation.

There are multiple electrode regions on the inner side of the lower body clothing portion 122, in areas corresponding to target body parts to be electrically stimulated, such as areas corresponding to the user's legs, buttocks, and other body parts. Each of the multiple electrodes is electrically connected to a second control unit connection part 20b by an individual electrical cable 36. The second control unit connection part 20b is provided at a position corresponding to the lower body control unit 126 shown in FIG. 4, and, when the lower body control unit 126 is attached to the second control unit connection part 20b, the second control unit connection part 20b and the lower body control unit 126 are electrically connected. Each of the multiple electrical cables 36 connected to an electrode is stretchable and twisted with a cable fixing tape 49, which is sewn onto the lower body clothing portion 122 at a position preceding the electrode, to be fixed in position; accordingly, even when the electrical cable 36 is pulled in the wake of stretching or contraction of the lower body clothing portion 122, the electrical cable 36 can be prevented from being disconnected from the electrode.

In an area corresponding to the front of the right thigh, as a pair of a cathode and an anode arranged vertically such that the right quadriceps muscle is positioned therebetween, an 11th electrode part 30k is provided in an upper part, and a 12th electrode part 301 is provided in a lower part. When a voltage is applied between the electrodes via an 11th electrical cable 36k and a 12th electrical cable 361 by the lower body control unit 126, electrical stimulation is provided to the right quadriceps muscle. The 11th electrical cable 36k is provided from the 11th electrode part 30k on the front of the right thigh through the crotch to be connected to a 13th electrode part 30m on the front of the left thigh, so as to be electrically connected to the second control unit connection part 20b via the 13th electrode part 30m. At a position preceding the 11th electrode part 30k, an 11th cable fixing tape 49k is sewn, and the 11th electrical cable 36k is twisted with the 11th cable fixing tape 49k such as to be fixed in position. The 12th electrical cable 361 is provided from the 12th electrode part 301 on the front of the right thigh through the crotch to be connected to a 14th electrode part 30n on the front of the left thigh, so as to be electrically connected to the second control unit connection part 20b via the 14th electrode part 30n. Also, at a position preceding the 12th electrode part 301, a 12th cable fixing tape 491 is sewn, and the 12th electrical cable 361 is twisted with the 12th cable fixing tape 491 such as to be fixed in position.

In an area corresponding to the front of the left thigh, as a pair of a cathode and an anode arranged vertically such that the left quadriceps muscle is positioned therebetween, the 13th electrode part 30m is provided in an upper part, and the 14th electrode part 30n is provided in a lower part. When a voltage is applied between the electrodes via a 13th electrical cable 36m and a 14th electrical cable 36n by the lower body control unit 126, electrical stimulation is provided to the left quadriceps muscle. At a position preceding the 13th electrode part 30m, a 13th cable fixing tape 49m is sewn, and the 13th electrical cable 36m, which connects the 13th electrode part 30m and the second control unit connection part 20b, and the 11th electrical cable 36k are twisted with the 13th cable fixing tape 49m such as to be fixed in position. Also, at a position preceding the 14th electrode part 30n, a 14th cable fixing tape 49n is sewn, and the 14th electrical cable 36n, which connects the 14th electrode part 30n and the second control unit connection part 20b, and the 12th electrical cable 361 are twisted with the 14th cable fixing tape 49n such as to be fixed in position.

In an area corresponding to the back of the right thigh, as a pair of a cathode and an anode arranged vertically such that the right hamstring muscles including the biceps femoris muscle are positioned therebetween, a 15th electrode part 30o is provided in an upper part, and a 16th electrode part 30p is provided in a lower part. When a voltage is applied between the electrodes via a 15th electrical cable 36o and a 16th electrical cable 36p by the lower body control unit 126, electrical stimulation is provided to the right hamstring muscles including the biceps femoris muscle. The 15th electrical cable 36o is provided from the 15th electrode part 30o on the back of the right thigh through the crotch to be connected to a 17th electrode part 30q on the back of the left thigh, so as to be electrically connected to the second control unit connection part 20b via the 17th electrode part 30q. At a position preceding the 15th electrode part 30o, a 15th cable fixing tape 49o is sewn, and the 15th electrical cable 36o is twisted with the 15th cable fixing tape 49o such as to be fixed in position. The 16th electrical cable 36p is provided from the 16th electrode part 30p on the back of the right thigh through the crotch to be connected to an 18th electrode part 30r on the back of the left thigh, so as to be electrically connected to the second control unit connection part 20b via the 18th electrode part 30r. Also, at a position preceding the 16th electrode part 30p, a 16th cable fixing tape 49p is sewn, and the 16th electrical cable 36p is twisted with the 16th cable fixing tape 49r such as to be fixed in position.

In an area corresponding to the back of the left thigh, as a pair of a cathode and an anode arranged vertically such that the left hamstring muscles including the biceps femoris muscle are positioned therebetween, the 17th electrode part 30q is provided in an upper part, and the 18th electrode part 30r is provided in a lower part. When a voltage is applied between the electrodes via a 17th electrical cable 36q and an 18th electrical cable 36r by the lower body control unit 126, electrical stimulation is provided to the left hamstring muscles including the biceps femoris muscle. At a position preceding the 17th electrode part 30q, a 17th cable fixing tape 49q is sewn, and the 17th electrical cable 36q, which connects the 17th electrode part 30q on the back of the left thigh and the second control unit connection part 20b on the front of the left thigh, and the 15th electrical cable 36o are twisted with the 17th cable fixing tape 49q such as to be fixed in position. Also, at a position preceding the 18th electrode part 30r, an 18th cable fixing tape 49r is sewn, and the 18th electrical cable 36r, which connects the 18th electrode part 30r on the back of the left thigh and the second control unit connection part 20b on the front of the left thigh, and the 16th electrical cable 36p are twisted with the 18th cable fixing tape 49r such as to be fixed in position.

In an area corresponding to the right buttock, as a pair of a cathode and an anode arranged vertically such that the right gluteus maximus and gluteus medius muscles are positioned therebetween, a 19th electrode part 30s is provided near the upper outer gluteus medius, and a 20th electrode part 30t is provided near the lower inner gluteus maximus. When a voltage is applied between the electrodes via a 19th electrical cable 36s and a 20th electrical cable 36t by the lower body control unit 126, electrical stimulation is provided to the right gluteus maximus and gluteus medius muscles. The 19th electrical cable 36s is provided from the 19th electrode part 30s on the right buttock through the center of the upper buttock to be connected to a 21st electrode part 30u on the left buttock, so as to be electrically connected to the second control unit connection part 20b via the 21st electrode part 30u. At a position preceding the 19th electrode part 30s, a 19th cable fixing tape 49s is sewn, and the 19th electrical cable 36s is twisted with the 19th cable fixing tape 49s such as to be fixed in position. The 20th electrical cable 36t is provided from the 20th electrode part 30t on the right buttock through the center of the upper buttock to be connected to a 22nd electrode part 30v on the left buttock, so as to be electrically connected to the second control unit connection part 20b via the 22nd electrode part 30v. Also, at a position preceding the 20th electrode part 30t, a 20th cable fixing tape 49t is sewn, and the 20th electrical cable 36t is twisted with the 20th cable fixing tape 49t such as to be fixed in position.

In an area corresponding to the left buttock, as a pair of a cathode and an anode arranged vertically such that the left gluteus maximus and gluteus medius muscles are positioned therebetween, the 21st electrode part 30u is provided near the upper outer gluteus medius, and the 22nd electrode part 30v is provided near the lower inner gluteus maximus. When a voltage is applied between the electrodes via a 21st electrical cable 36u and a 22nd electrical cable 36v by the lower body control unit 126, electrical stimulation is provided to the left gluteus maximus and gluteus medius muscles. At a position preceding the 21st electrode part 30u, a 21st cable fixing tape 49u is sewn, and the 21st electrical cable 36u, which connects the 21st electrode part 30u on the left gluteus medius muscle and the second control unit connection part 20b on the front of the left thigh, and the 19th electrical cable 36s are twisted with the 21st cable fixing tape 49u such as to be fixed in position. Also, at a position preceding the 22nd electrode part 30v, a 22nd cable fixing tape 49v is sewn, and the 22nd electrical cable 36v, which connects the 22nd electrode part 30v on the left gluteus maximus muscle and the second control unit connection part 20b on the front of the left thigh, and the 20th electrical cable 36t are twisted with the 22nd cable fixing tape 49v such as to be fixed in position.

A cable fixing tape 59b is provided to fix the 11th electrical cable 36k connecting the 11th electrode part 30k and the 13th electrode part 30m, the 12th electrical cable 361 connecting the 12th electrode part 301 and the 14th electrode part 30n, the 15th electrical cable 36o connecting the 15th electrode part 30o and the 17th electrode part 30q, and the 16th electrical cable 36p connecting the 16th electrode part 30p and the 18th electrode part 30r. The cable fixing tape 59b is sewn near the crotch onto the fabric of the lower body clothing portion 122. Thus, the four electrical cables 36 are bundled by the cable fixing tape 59b and fixed near the crotch. Also, a cable fixing tape 59c is provided to fix the 19th electrical cable 36s connecting the 19th electrode part 30s and the 21st electrode part 30u, and the 20th electrical cable 36t connecting the 20th electrode part 30t and the 22nd electrode part 30v. The cable fixing tape 59c is sewn near the center of the upper buttock onto the fabric of the lower body clothing portion 122. Thus, the two electrical cables 36 are bundled by the cable fixing tape 59c and fixed near the center of the upper buttock.

With the arrangement and wiring as described above, the electrical cables can be provided without exposure to the outside. Also, since the configurations other than the lower body control unit 126 are water-resistant, only by removing the lower body control unit 126, the fitness wear 102 can be washed without removing the electrode parts 30 and the electrical cables 36. Although FIGS. 6 illustrate an example of the lower body clothing portion 122 in which the electrodes are arranged also mainly for men, the arrangement and the size of the electrodes, for example, may be different for women.

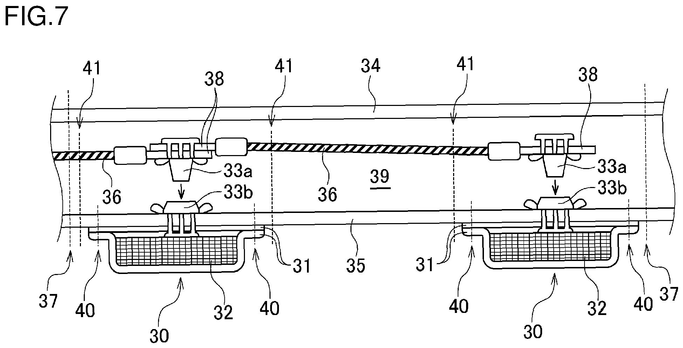

FIG. 7 is a partially enlarged view that illustrates arrangement and structures of a clothing portion, electrodes, and electrical cables. At least part of each of the upper body clothing portion 120 and the lower body clothing portion 122 has a multi-layered structure in which an outer fabric 34 and an inner fabric 35 overlap each other. In a predetermined region of the inner fabric 35, an electrode part 30 of which at least the outer surface is formed of a conductive polymer fabric 31 is provided. Multiple electrode parts 30 are provided at positions that respectively face multiple target body parts such as to be respectively in proximity to the multiple target body parts, so as to provide electrical stimulation to the multiple target body parts. Each electrode part 30 is configured to include a pad member 32 provided on the inner fabric 35 of the upper body clothing portion 120 or the lower body clothing portion 122, on the side facing the skin of the wearer, and the conductive polymer fabric 31 that covers the pad member 32. The pad member 32 may be formed of rectangular foam rubber, for example, and the pad member 32 forms a convex shape, which is the overall shape of the electrode part 30. The conductive polymer fabric 31 is knitted from conductive fibers formed by attaching and impregnating conductive polymers, such as PEDOT-pTS (poly(3,4-ethylenedioxythiophene)-p-toluenesulfonic acid) and PEDOT-PSS (poly(3,4-ethylenedioxythiophene)-polystyrene sulfonic acid), to or into fibers such as silk or synthetic fibers such as nylon. The conductive polymer fabric 31 does not include metal as a material and hence has high washing durability. Particularly, if conductive fibers with metal such as silver attached thereto or included therein are used, the conductivity may decrease due to oxidation or corrosion, and metal allergies may be caused depending on the users' constitutions; however, the conductive polymer fabric 31 has no such concern. Also, if conductive fibers with metal attached thereto are used, the conductivity may decrease due to peeling caused by friction, but the conductive polymer fabric 31 has no such concern. On the peripheral edges of the conductive polymer fabric 31 covering the pad member 32, edge sewing such as merrowing is applied to prevent fraying thereof, and the conductive polymer fabric 31 is sewn onto the inner fabric 35 at an electrode sewing part 40 as shown in FIG. 7. With electrodes formed of the conductive polymer fabric 31, a current can be applied between the electrodes without gel pads as attached to the electrodes of conventional electrical muscle stimulation devices. Also, by wetting the electrodes using a spray or the like such that the electrodes lightly retain water, the state of current conduction between the electrodes can be further stabilized even at the start of exercise when the user is not yet sweating. The conductivity may be further stabilized by raising the fibers of the conductive polymer fabric 31.

To the multiple electrode parts 30, electrical cables 36 are connected via electrode connection parts 33. For example, multiple electrode parts 30, which are anodes or cathodes, are connected by electrical cables 36, and electrode parts 30 and a control unit connection part 20 are also connected by electrical cables 36. An electrical cable 36 is connected to a conductive polymer fabric 31 via an electrode connection part 33, which penetrates the inner fabric 35 to electrically connect the outer side and inner side thereof. The electrode connection part 33 may be a metal clasp such as a snap button that can be electrically connected by fitting a convex electrode connection part 33a into a concave electrode connection part 33b, for example. The convex electrode connection part 33a is attached such as to catch a crimp terminal 38 formed at an end of an electrical cable 36. The concave electrode connection part 33b is attached such as to catch the conductive polymer fabric 31 and the inner fabric 35 and penetrate the inner fabric 35 at a position in contact with the pad member 32 within an electrode part 30, thereby electrically connecting the outer side and inner side of the inner fabric 35. When the convex electrode connection part 33a is fitted into the concave electrode connection part 33b, the electrical cable 36 is electrically connected to the conductive polymer fabric 31. Each electrical cable 36 is twisted with a cable fixing tape 49 provided at a cable fixing position 41, for example, to be fixed in position.

At least part of each electrical cable 36 and the connection portion between the electrical cable 36 and an electrode connection part 33 are placed within a closed space formed by sewing the outer fabric 34 and the inner fabric 35 in at least part of the vicinity of the part of the electrical cable 36 and the connection portion. For example, by sewing the outer fabric 34 and the inner fabric 35 at the positions of inter-fabric sewing parts 37 shown in FIG. 7, a closed space 39 is formed. In FIG. 7, the outer fabric 34 and the inner fabric 35 are illustrated far apart for the purpose of explaining the structure. However, the outer fabric 34 and the inner fabric 35 are actually located more closely and sewn at the positions of the inter-fabric sewing parts 37 to form the closed space 39. Also, the "closed" in the "closed space" does not necessarily mean being completely sealed, and it may be sufficient as long as the space is appropriately closed with the circumference thereof sewn such that at least the connection portions between the electrical cables 36 and the electrode connection parts 33 and the electrical cables 36 are not easily exposed to the outside. Around such a "closed space", a maintenance hole may be provided, through which the electrical cables 36 can be inserted and removed. With the structure described above, each of the electrodes of the first electrode part 30a through the 22nd electrode part 30v is disposed in a corresponding region of the fitness wear 102.

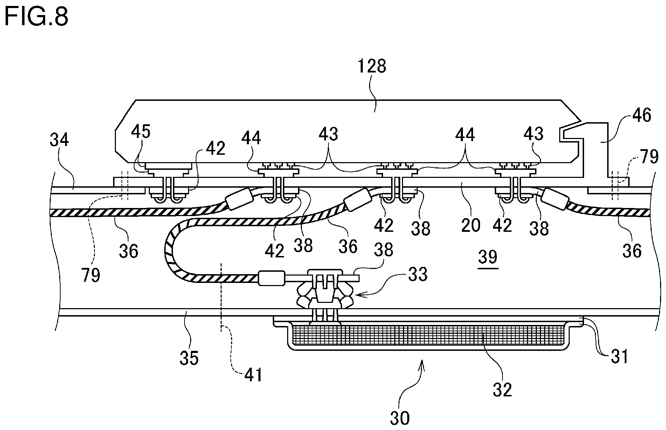

FIG. 8 is a partially enlarged sectional view that illustrates arrangement and structures of a clothing portion, an electrode, electrical cables, a control unit, and a control unit connection part. The structure of an electrode part 30 is the same as that in FIG. 7. With reference to FIG. 8, the connection between a control unit 128 and a control unit connection part 20 and the connection between the control unit connection part 20 and an electrode part 30 will be further described. The control unit 128 is the upper body control unit 124 or the lower body control unit 126 and attached to the control unit connection part 20. The control unit connection part 20 is configured such that a convex clasp 46 such as a snap fit component is formed at an end of a substrate made of resin, and the convex clasp 46 is fitted into a recess formed at an end of the control unit 128 so as to fix one end side of the control unit 128 to the control unit connection part 20. The other end side of the control unit 128 is also fixed to the control unit connection part 20 by means of a clasp such as a magnetic button 45. The both ends of the control unit connection part 20 are sewn onto the outer fabric 34 at the positions of flat plate sewing parts 79.

A pad connector 44 has a planar metal head part and leg parts protruding from the head part, and the leg parts penetrate through holes provided respectively in the control unit connection part 20, the crimp terminal 38 of an electrical cable 36, and a washer 42. In the state of catching the control unit connection part 20, crimp terminal 38, and washer 42, the pad connector 44 is fixed to the control unit connection part 20. This electrically connects the outer side and inner side of the control unit connection part 20. On the bottom surface of the control unit 128, multiple spring connectors 43, which are metal elastic contacts, are provided in multiple regions corresponding to the electrode connection parts 33. With the spring connectors 43 making pressure contact with the planer head parts of the pad connectors 44, the control unit 128 is attached and fixed to the control unit connection part 20. Accordingly, a current is applied from a power supply provided in the control unit 128 to the electrical cables 36 via the spring connectors 43 and the pad connectors 44, and a voltage is applied from the electrical cables 36 to the multiple electrode parts 30 via the electrode connection parts 33. Each electrical cable 36 is twisted with a cable fixing tape 49 provided at a cable fixing position 41 around an electrode part 30, for example, to be fixed in position.

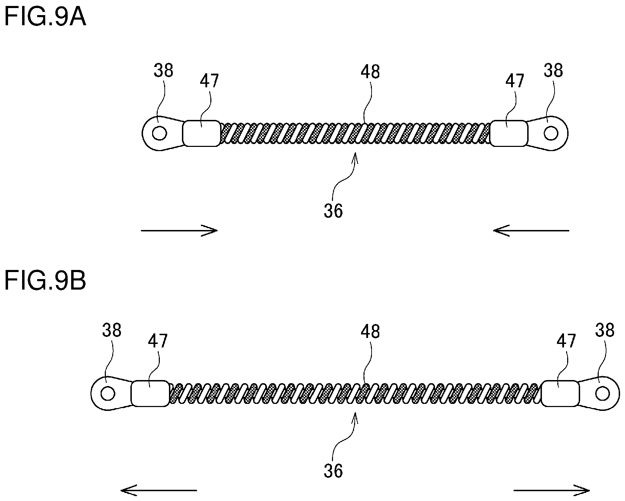

FIGS. 9 are external views of an electrical cable. Each electrical cable 36 is a stretchable electric wire and constituted by a stretchable cable part 48, and resin molds 47 and the crimp terminals 38 provided at the both ends. Each crimp terminal 38 is a ring-shaped flat metal terminal, and, through a hole in the center thereof, an electrode connection part 33a or the leg parts of a pad connector 44 are made to pass such that the crimp terminal 38 is electrically connected thereto. The stretchable cable part 48 is configured such that multiple coated conductive wires, formed by coating core wires with resin, and multiple resin wire rods are alternately wound spirally around a stretchable elastic core material made of resin. Each end of the coated conductive wires is connected to a crimp terminal 38, and the connection part therebetween is covered with a resin mold 47. The elastic core material has elasticity to return to its original state when bent. FIG. 9A schematically illustrates a contracted state of an electrical cable 36, and FIG. 9B schematically illustrates a stretched state of an electrical cable 36. In the stretched state of the electrical cable 36, gaps between the spirally-wound coated conductive wires are widened, and the elastic core material can be partially exposed to the outside through the gaps. In each electrical cable 36, the elastic core material with the coated conductive wires and the resin wire rods wound spirally therearound are stretchable in a longitudinal direction of the elastic core material, and the entire length thereof can be extended up to about 1.4 times, for example. Thus, the electrical cables 36 each have a higher stretching rate than the fabric of the fitness wear 102, which has relatively high stretchability. Therefore, even when the fitness wear 102 is stretched due to the body shape and movement of the user and also even when the electrical cables 36 are pulled in the wake of the stretching, such pull can be sufficiently absorbed by the stretching of the electrical cables 36, and the electrical cables 36 can be prevented from being damaged or from being disconnected from the electrode parts 30 or the like. Inside each resin mold 47, a crimp terminal 38 and a stretchable cable part 48 are prevented from coming off by means of an adhesive, which also serves as waterproof treatment to prevent water from entering the stretchable cable part 48. The core wires within the coated conductive wires of the stretchable cable part 48 are soldered to the crimp terminals 38. Since the electrical cables 36 have water resistance, they can be washed without being removed from the fitness wear 102. Therefore, the electrical cables 36 can be placed within the closed space 39 formed between the outer fabric 34 and the inner fabric 35 without being exposed to the outside basically, which does not spoil the aesthetics of the fitness wear 102.

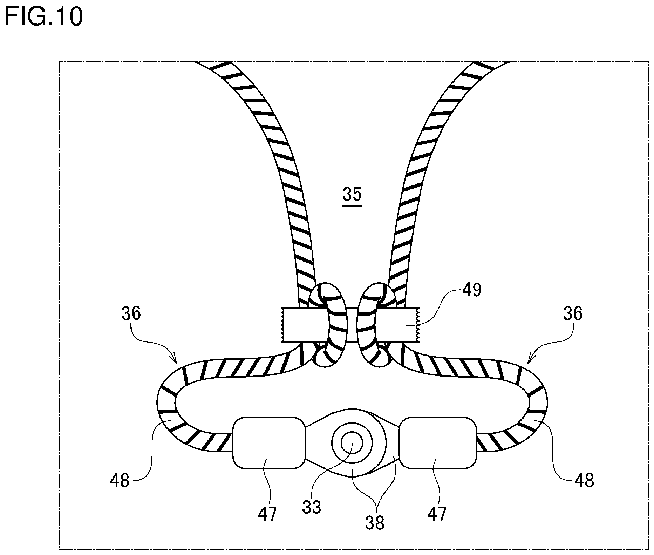

FIG. 10 is a partially enlarged view that illustrates a connection portion and a sewing portion of electrical cables 36 on the inner fabric 35. With the crimp terminal 38 at an end of an electrical cable 36 caught by an electrode connection part 33, the electrical cable 36 is attached to the inner side of an electrode part 30 on the inner fabric 35. Near the electrode connection part 33, a cable fixing tape 49, which is a non-stretchable tape-like synthetic fiber, is disposed, and the both ends thereof are sewn onto the inner fabric 35. An electrical cable 36 is made to pass through and twisted with a cable fixing tape 49, with a margin (play) left in the cable length by detouring the cable, compared to the shortest distance from a corresponding electrode connection part 33 to the cable fixing tape 49. The multiple electrical cables 36 are provided such that, between an electrode part 30 and a control unit connection part 20 or between electrode parts 30, an electrical cable 36 is linearly provided overall so that there is little play, whereas, around an electrode part 30, i.e., between an electrode connection part 33 and a cable fixing tape 49, an electrical cable 36 is provided with a margin or play left therein. Even when the electrical cables 36 are pulled in the wake of stretching of the fitness wear 102 while the user moves his or her body, such pull can be absorbed by the stretchability of the electrical cables 36 and can also be stopped at the cable fixing tapes 49. Also, even if the cable fixing tapes 49 themselves are pulled a little, the pull can be absorbed by the margin (play) of the electrical cables 36. Accordingly, the crimp terminals 38 and the electrode connection parts 33 are not subjected to more load than necessary, and the electrical cables 36 can be prevented from coming off the connection portions.

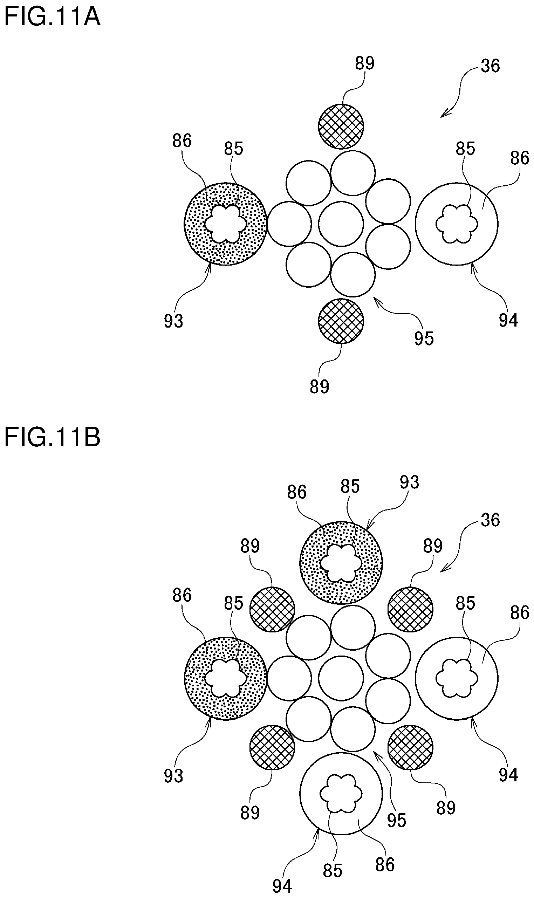

FIGS. 11 are explanatory sectional views that each schematically illustrate a configuration of an electrical cable 36. Since FIGS. 11 are drawn merely to conceptually explain each configuration in an electrical cable 36, cross-sectional shapes are illustrated conveniently and different from the actual shapes. FIG. 11A illustrates an electrical cable 36 in the present embodiment, which mainly includes one first coated conductive wire 93 and one second coated conductive wire 94. Each of the first coated conductive wire 93 and the second coated conductive wire 94 is constituted by a conductive wire 85, and insulation coating 86 that coats the conductive wire 85 with resin. An elastic core material 95 is stretchable twisted yarn formed by twisting multiple rubber threads. Fixing yarn 89 is thinner than the first coated conductive wire 93 and the second coated conductive wire 94 and is, for example, twisted yarn formed by twisting multiple threads of synthetic fiber, such as nylon. Around the elastic core material 95, the first coated conductive wire 93 and the second coated conductive wire 94 are spirally wound in the same winding direction to be arranged alternately. Also, the first coated conductive wire 93 and the second coated conductive wire 94 are wound with the fixing yarn 89 provided therebetween so as to avoid damage to the first coated conductive wire 93 and the second coated conductive wire 94 caused by contact therebetween during stretching and contraction. Further, to facilitate maintaining the wound state of the first coated conductive wire 93 and the second coated conductive wire 94, the fixing yarn 89 is wound while being interwoven in a direction that intersects the winding direction of the first coated conductive wire 93 and the second coated conductive wire 94. Thus, the electrical cable 36 is formed.

FIG. 11B illustrates an electrical cable 36 in a modification, which mainly includes two first coated conductive wires 93 and two second coated conductive wires 94. Around the elastic core material 95, the two first coated conductive wires 93 and the two second coated conductive wires 94 are alternately wound spirally, with four strands of the fixing yarn 89 provided therebetween and interwoven, so as to form the electrical cable 36.

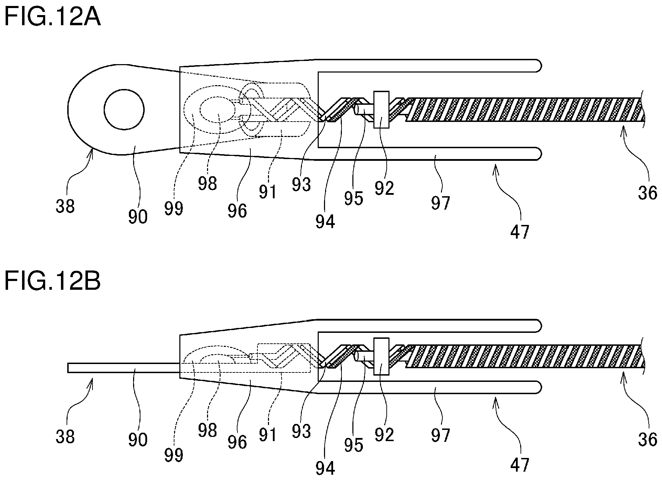

FIGS. 12 are explanatory sectional views that each schematically illustrate an internal structure of a resin mold 47. FIG. 12A is a sectional view viewed from the above, and FIG. 12B is a sectional view viewed from a side. Since FIGS. 12 are drawn merely to provide conceptual explanation, including the internal structure that does not appear on the external appearance, the parts that do not appear on the external appearance are also illustrated with solid lines for the sake of convenience. The crimp terminal 38 is a round bare crimp terminal constituted by a flat plate part 90 of racket shape of which one end is formed in a substantial ring, and an electric wire fixing part 91 of cylindrical shape provided at the other end of the flat plate part 90. The first coated conductive wire 93 and the second coated conductive wire 94 of an electrical cable 36 are made to pass through the electric wire fixing part 91, and the core wires of the first coated conductive wire 93 and the second coated conductive wire 94 are soldered to the flat plate part 90 (a solder joint part 98) and fixed by means of an adhesive applied over the solder joint part 98 (a fixed part 99). The resin mold 47 is constituted by a seal molding portion 96, which is resin molded to seal the fixed part 99, the end parts of the first coated conductive wire 93 and the second coated conductive wire 94, and the electric wire fixing part 91, and by a hollow molding portion 97 of cylindrical shape that contains a portion of the end part of the electrical cable 36 that is not sealed by the seal molding portion 96. Accordingly, the resin mold 47 is formed as a whole in the shape of a pen cap.

Here, it does not mean that, in the configurations included in the electrical cable 36, the first coated conductive wire 93 and the second coated conductive wire 94 together with the elastic core material 95 are made to pass through the electric wire fixing part 91 to be fixed and sealed. Instead, only the first coated conductive wire 93 and the second coated conductive wire 94 are made to pass through the electric wire fixing part 91 to be fixed and sealed. More specifically, the elastic core material 95 is cut at a position before it reaches the seal molding portion 96 or the electric wire fixing part 91, and the relative positions of the elastic core material 95, the first coated conductive wire 93, and the second coated conductive wire 94 are fixed around the cut position by a core material fixing part 92. The core material fixing part 92 is a metal fixture for preventing the elastic core material 95, the first coated conductive wire 93, and the second coated conductive wire 94 from shifting. The core material fixing part 92 is not fixed to the resin mold 47 and can move freely inside the hollow molding portion 97. The core material fixing part 92 is provided to fix the elastic core material 95 to the first coated conductive wire 93 and the second coated conductive wire 94, and the core material fixing part 92 can only move freely together with the electrical cable 36 connected therewith inside the hollow molding portion 97 along with the stretching and contraction of the first coated conductive wire 93 and the second coated conductive wire 94 present between the core material fixing part 92 and the seal molding portion 96. Although the elastic core material 95 does not extend from the core material fixing part 92 to the seal molding portion 96, the first coated conductive wire 93 and the second coated conductive wire 94 present therebetween are housed and protected in the hollow molding portion 97 and hence can be prevented from being damaged by external bending stress. As illustrated in FIG. 10, since each electrical cable 36 is twisted with a cable fixing tape 49 near an electrode part 30 to hold the tensile stress, the first coated conductive wire 93 and the second coated conductive wire 94 between the core material fixing part 92 and the seal molding portion 96 can be prevented from being damaged by excessive tensile stress.

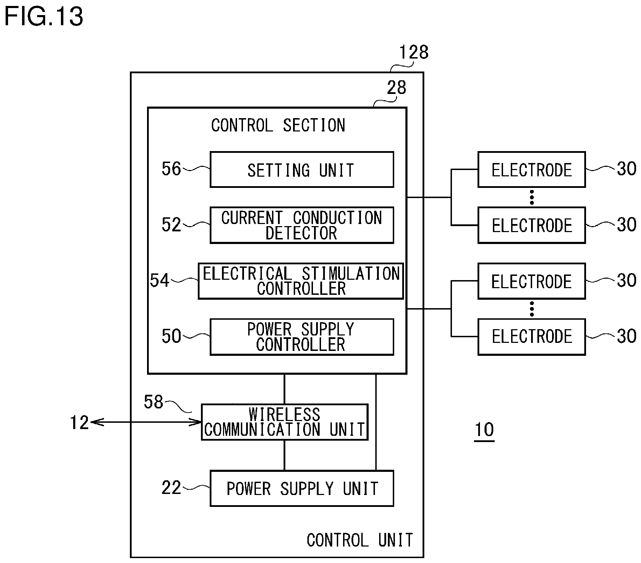

FIG. 13 is a block diagram that illustrates a functional configuration of the control unit. The control unit 128 includes a power supply unit 22, a control section 28, and a wireless communication unit 58. The power supply unit 22, control section 28, and wireless communication unit 58 are housed in a casing having a shape attachable to a control unit connection part 20. The control section 28 includes a power supply controller 50, a current conduction detector 52, an electrical stimulation controller 54, and a setting unit 56. As the control unit 128, there are the upper body control unit 124 attached to the upper body clothing portion 120, and the lower body control unit 126 attached to the lower body clothing portion 122, which both have the same configuration. As a modification, there may be a difference in configuration or shape between the upper body control unit 124 and the lower body control unit 126.

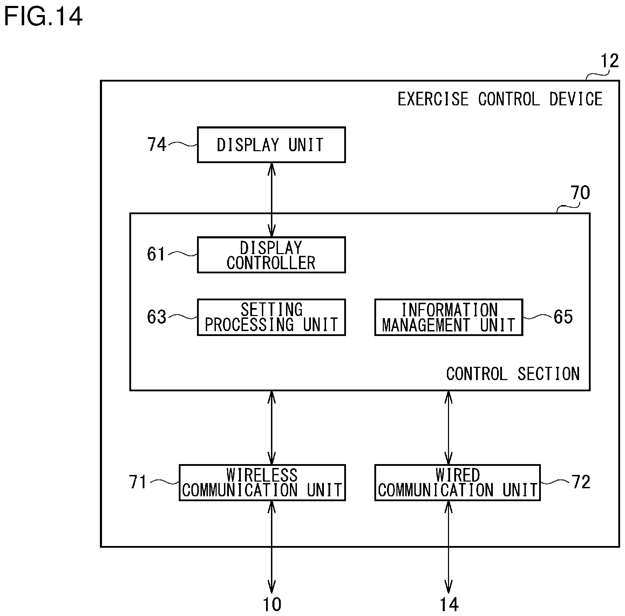

Each block in the control section 28 can be implemented by an element such as an integrated circuit or by a mechanism in terms of hardware, and by a computer program or the like in terms of software. FIG. 13 illustrates functional blocks implemented by the cooperation of those components. Therefore, it will be understood by those skilled in the art, who have found the present specification, that these functional blocks may be implemented in a variety of forms by combinations of hardware and software. The same applies to each block in FIG. 14.

The power supply unit 22 is a secondary battery, such as a lithium-ion battery, but may also be a replaceable primary battery. The power supply unit 22 is electrically connected to a wireless communication module as the wireless communication unit 58 and a control circuit as the control section 28 and supplies electricity to each of them. The control unit 128 may be provided with a power button, and the power supply unit 22 may be turned on and off in response to the operation of the power button.

The power supply controller 50 controls the charging of the power supply unit 22 and transmits information indicating the charging status to a corresponding exercise control device 12 via the wireless communication unit 58. The current conduction detector 52 detects a resistance value between anode and cathode electrodes in order to detect whether an electric current can be conducted between the electrodes. For example, the current conduction detector 52 of the upper body control unit 124 detects a resistance value between the first electrode part 30a and the second electrode part 30b for the abdominal muscles, a resistance value between the third electrode part 30c and the fourth electrode part 30d for the right flank, a resistance value between the fifth electrode part 30e and the sixth electrode part 30f for the left flank, a resistance value between the seventh electrode part 30g and the eighth electrode part 30h for the right arm, and a resistance value between the ninth electrode part 30i and the tenth electrode part 30j for the left arm. Similarly, the current conduction detector 52 of the lower body control unit 126 detects a resistance value between the 11th electrode part 30k and the 12th electrode part 301 for the front of the right thigh, a resistance value between the 13th electrode part 30m and the 14th electrode part 30n for the front of the left thigh, a resistance value between the 15th electrode part 30o and the 16th electrode part 30p for the back of the right thigh, a resistance value between the 17th electrode part 30q and the 18th electrode part 30r for the back of the left thigh, a resistance value between the 19th electrode part 30s and the 20th electrode part 30t for the right buttock, and a resistance value between the 21st electrode part 30u and the 22nd electrode part 30v for the left buttock. The current conduction detector 52 judges that current conduction is possible when the detected resistance value is less than a threshold and judges that current conduction is impossible when the detected resistance value is greater than or equal to the threshold.

When the current conduction detector 52 has judged that the current conduction is possible, the electrical stimulation controller 54 applies a set voltage between the anode and cathode electrodes for a predetermined operation time (such as 10 minutes) with a predetermined period (such as a period where the frequency is 20 Hz). Thus, electrical stimulation can be applied to the areas where electrodes are provided, such as the user's abdominal muscles, flanks, arms, legs, and buttocks. The setting unit 56 sets a voltage value and an increase or a decrease thereof controlled by the electrical stimulation controller 54 according to a predetermined exercise control program. The set voltage value may be set to any of 100 levels of intensity, for example. There are two modes of exercise control programs: in the first mode, which aims at muscle hypertrophy, electrical stimulation is applied mainly at a frequency of 20 Hz; in the second mode, which aims at aerobic exercise, electrical stimulation is applied mainly at a frequency lower than 20 Hz (such as 4 to 10 Hz). The first mode is used for resistance training in which, basically at a frequency of 20 Hz, for example, involuntary movement produced by electrical stimulation from the fitness wear 102 is mainly performed, and voluntary movement only with slow movement is added for the purpose of muscle hypertrophy. Meanwhile, the second mode is used for cardio training as hybrid training in which, basically at a frequency of 4 Hz, for example, voluntary movement such as bike training is added to involuntary movement produced by electrical stimulation from the fitness wear 102. The cardio training includes anaerobic exercise with speedy motion and aerobic exercise aiming at fat burning through calorie consumption. The setting unit 56 retains, besides an exercise control program that operates based on instructions received from the wireless communication unit 58, an exercise control program that runs independently, of which the execution can also be started by operating a remote control unit (an information terminal such as a cellular phone and a control application installed thereon, for example) connected via wireless communication.

The wireless communication unit 58 receives information, such as the set voltage, from a corresponding exercise control device 12 via short-range wireless communication and transmits the information to the setting unit 56. When the wireless communication unit 58 has received, from the exercise control device 12, information on the set voltage value or information on an instruction to increase or decrease the set voltage value, the setting unit 56 increases or decreases the set voltage value to be applied, based on the information thus received. For example, during the operation of setting the voltage value before the start of exercise, every time the set voltage value is increased or decreased, the electrical stimulation controller 54 applies a voltage between the electrodes at the newly set voltage value, so as to allow the user to feel and confirm the set voltage value, or exercise intensity, newly set. Even during exercise, i.e., during voltage application, when the wireless communication unit 58 has received from the exercise control device 12 an instruction to increase or decrease the exercise intensity, the wireless communication unit 58 transmits the information on the increased or decreased set voltage to the setting unit 56, which then increases or decreases the set voltage value accordingly. However, if the wireless communication unit 58 has received an instruction to increase or decrease the exercise intensity from a device other than the exercise control device 12 to which the wireless communication unit 58 is connecting, the wireless communication unit 58 will ignore the instruction and will not follow an instruction to increase or decrease the exercise intensity from other devices. This is to prevent the voltage value from being increased or decreased by a person other than the user without permission. The wireless communication unit 58 may transmit, to the exercise control device 12, information indicating the state of voltage application by the electrical stimulation controller 54, i.e., the state of exercise execution. The control unit 128 may be provided with a pairing button, and, when the pairing button is turned on, the wireless communication unit 58 may establish wireless communication connection. As a modification, the wireless communication unit 58 may connect with another wireless communication unit 58 included in another control unit by pairing via short-range wireless communication. More specifically, the wireless communication unit 58 of the upper body control unit 124 and the wireless communication unit 58 of the lower body control unit 126 may be wirelessly connected and may be linked to each other through transfer of information, including the set voltage value, from one to the other. In this case, if a voltage value is set in the setting unit 56 of the upper body control unit 124, for example, the set voltage value will be transferred from the wireless communication unit 58 of the upper body control unit 124 to the wireless communication unit 58 of the lower body control unit 126.

FIG. 14 is a block diagram that illustrates a functional configuration of an exercise control device 12. The exercise control device 12 includes a control section 70, a wireless communication unit 71, a wired communication unit 72, and a display unit 74. The control section 70 includes a display controller 61, a setting processing unit 63, and an information management unit 65. Each of the first and second exercise programs is implemented by an exercise control program that controls a voltage by means of the control unit 128 and also controls video display linked to such voltage control. Each of the third and fourth exercise programs is implemented by an exercise control program that controls a voltage while synchronizing multiple control units 128 of multiple users by allowing the exercise control device 12 to transmit a control signal such as a start signal simultaneously to the multiple control units 128 and that also controls video display linked to such voltage control. Each exercise program is performed by each user moving his or her own body following an example video displayed on a display device 106.

The wireless communication unit 71 transmits and receives information to and from a control unit 128 via short-range wireless communication. To support multiple users at the same time, the wireless communication unit 71 needs to simultaneously communicate with at least as many control units 128 as the multiple users. Accordingly, one-way broadcast communication will be mainly used, and, if necessary, bidirectional communication by pairing will also be used. In the case of bidirectional communication, information received from each control unit 128 may include individual identification information used for user registration and individual management, and a unique network address (MAC address). Also, by receiving information from the control units 128, the multiple sets of fitness wear 102 of the multiple users being powered on can be recognized. The display unit 74 is a touch-panel display device, such as a liquid crystal panel and an organic EL panel, and displays information on the screen and accepts operational input from a user or an instructor.

Based on the operational input from a user or an instructor to the display unit 74, the setting processing unit 63 sets the content of control performed by a control unit 128. When supporting multiple users at the same time, the setting processing unit 63 sets the content of control performed by each of multiple control units 128 for the respective users. The setting processing unit 63 may be capable of setting the content of control separately for each of multiple body parts. As the content of control, the setting processing unit 63 sets the intensity of electrical stimulation, such as a voltage value of one of 100 levels, applied by an electrical muscle stimulation device 10. The setting processing unit 63 then transmits information indicating the content of control to every control unit 128 via the wireless communication unit 71 by broadcast communication, so as to control the exercise provided by the control unit 128. For example, the setting processing unit 63 transmits, to a control unit 128, an exercise start signal, a pause signal, an end signal, a voltage value signal, and the like to control the control unit 128. Meanwhile, in order to reduce battery consumption by communication in each control unit 128, minimizing communication is preferable in design. For example, a voltage value set for a control unit 128 and data of exercise control programs may be stored in the information management unit 65, so that the voltage value information is not acquired from the control unit 128. Also, it may be judged that an exercise program is completed after a predetermined period of time elapses, without transmission of the end signal for the exercise.

The display controller 61 displays, on the display unit 74, a list of contents related to the execution status of an exercise program, the setting status and the operation status of an electrical muscle stimulation device 10, and the like. The list thus displayed may include a user in the personal space 81 connected via communication. The display controller 61 transmits, via the wired communication unit 72, data regarding the execution status of an exercise program to a display control device 104 in order to display an example video or the like on a corresponding display device 106 based on the exercise program. Also, the display controller 61 displays, on the display device 106 via the display control device 104, an image of a human body model and also a video that dynamically shows the rhythm of the voltage pulse and the movement of muscles during exercise based on the control state of the corresponding electrical muscle stimulation device 10.

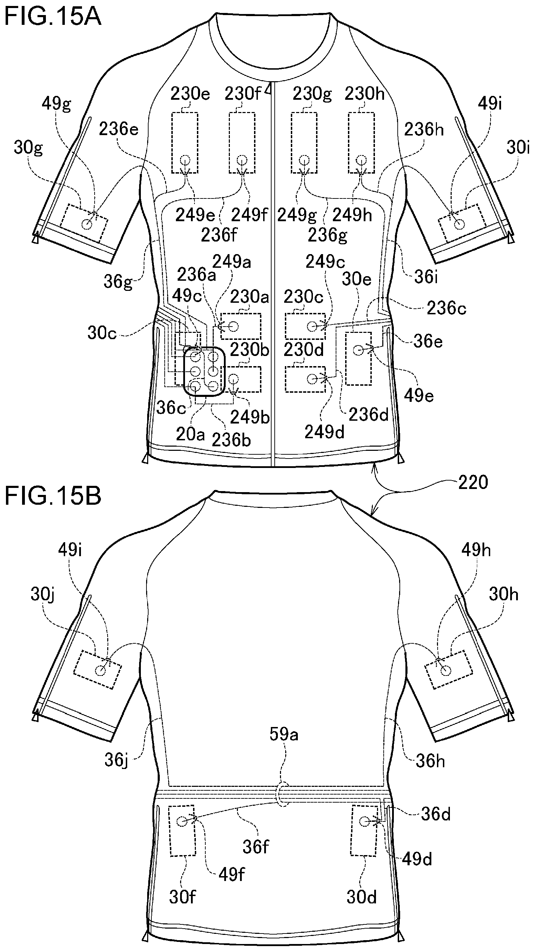

FIGS. 15 illustrate another example of the upper body clothing portion. An upper body clothing portion 220 in FIGS. 15 differs from the upper body clothing portion 120 in FIGS. 5 in configuration and arrangement of some electrodes and in arrangement of electrical cables 36 and cable fixing tapes 49 associated therewith. First, as the electrodes for the rectus abdominis muscles, instead of the first electrode part 30a and the second electrode part 30b of the upper body clothing portion 120 in FIGS. 5 arranged on the left and right rectus abdominis muscles to apply electrical stimulation in a horizontal direction, two electrodes are vertically arranged on each of the left and right rectus abdominis muscles to apply electrical stimulation in a vertical direction in the upper body clothing portion 220 of FIGS. 15. More specifically, in an area corresponding to the right rectus abdominis muscle, as a pair of a cathode and an anode arranged vertically such that the right rectus abdominis muscle is positioned therebetween, a first electrode part 230a is provided in an upper part, and a second electrode part 230b is provided in a lower part. Accordingly, a voltage is applied between the electrodes via a first electrical cable 236a and a second electrical cable 236b by the upper body control unit 124. At a position preceding the first electrode part 230a, a first cable fixing tape 249a is sewn, and the first electrical cable 236a, which connects the first electrode part 230a and the first control unit connection part 20a, is twisted with the first cable fixing tape 249a such as to be fixed in position. Also, at a position preceding the second electrode part 230b, a second cable fixing tape 249b is sewn, and the second electrical cable 236b, which connects the second electrode part 230b and the first control unit connection part 20a, is twisted with the second cable fixing tape 249b such as to be fixed in position. Similarly, in an area corresponding to the left rectus abdominis muscle, as a pair of a cathode and an anode arranged vertically such that the left rectus abdominis muscle is positioned therebetween, a third electrode part 230c is provided in an upper part, and a fourth electrode part 230d is provided in a lower part. Accordingly, a voltage is applied between the electrodes via a third electrical cable 236c and a fourth electrical cable 236d by the upper body control unit 124. At a position preceding the third electrode part 230c, a third cable fixing tape 249c is sewn, and the third electrical cable 236c, which is provided from the third electrode part 230c on the left abdominal muscle to the first control unit connection part 20a on the front side through the left flank, the back, and the right flank, is twisted with the third cable fixing tape 249c such as to be fixed in position. Also, at a position preceding the fourth electrode part 230d, a fourth cable fixing tape 249d is sewn, and the fourth electrical cable 236d, which is provided from the fourth electrode part 230d on the left abdominal muscle to the first control unit connection part 20a on the front side through the left flank, the back, and the right flank, is twisted with the fourth cable fixing tape 249d such as to be fixed in position.

In the upper body clothing portion 220 of FIGS. 15, two electrodes are further arranged horizontally on each of the left and right pectoralis major muscles to apply electrical stimulation in a horizontal direction. More specifically, in an area corresponding to the right pectoralis major muscle, as a pair of a cathode and an anode arranged horizontally such that the right pectoralis major muscle is positioned therebetween, a fifth electrode part 230e is provided on the right, and a sixth electrode part 230f is provided on the left. Accordingly, a voltage is applied between the electrodes via a fifth electrical cable 236e and a sixth electrical cable 236f by the upper body control unit 124. At a position preceding the fifth electrode part 230e, a fifth cable fixing tape 249e is sewn, and the fifth electrical cable 236e, which connects the fifth electrode part 230e and the first control unit connection part 20a, is twisted with the fifth cable fixing tape 249e such as to be fixed in position. Also, at a position preceding the sixth electrode part 230f, a sixth cable fixing tape 249f is sewn, and the sixth electrical cable 236f, which connects the sixth electrode part 230f and the first control unit connection part 20a, is twisted with the sixth cable fixing tape 249f such as to be fixed in position. Similarly, in an area corresponding to the left pectoralis major muscle, as a pair of a cathode and an anode arranged horizontally such that the left pectoralis major muscle is positioned therebetween, a seventh electrode part 230g is provided on the right, and an eighth electrode part 230h is provided on the left. Accordingly, a voltage is applied between the electrodes via a seventh electrical cable 236g and an eighth electrical cable 236h by the upper body control unit 124. At a position preceding the seventh electrode part 230g, a seventh cable fixing tape 249g is sewn, and the seventh electrical cable 236g, which is provided from the seventh electrode part 230g on the left pectoralis major muscle to the first control unit connection part 20a on the front side through the left flank, the back, and the right flank, is twisted with the seventh cable fixing tape 249g such as to be fixed in position. Also, at a position preceding the eighth electrode part 230h, an eighth cable fixing tape 249h is sewn, and the eighth electrical cable 236h, which is provided from the eighth electrode part 230h on the left pectoralis major muscle to the first control unit connection part 20a on the front side through the left flank, the back, and the right flank, is twisted with the eighth cable fixing tape 249h such as to be fixed in position.

The fitness wear 102 as described above can also be used in hybrid training that combines involuntary movement produced by the electrical muscle stimulation device 10 with voluntary movement produced by consciously moving the body in bike training using a fitness bike or a spin bike, for example. In the first and second exercise programs, bike training may be performed by each user, with a single exercise control device 12 linked to a single bike. Also, in the third and fourth exercise programs, bike training may be performed by multiple users watching the same video and following the same program, as group training in which a single exercise control device 12 is linked to multiple bikes. The exercise program in the hybrid training combined with the bike training is a program of pedaling a bike while producing twitching of muscles with a frequency of 4 Hz, for example, and the duration of the program may be 10 to 30 minutes, for example.

In the bike training, the knees and thighs are raised high in pedaling. However, a first control unit (the upper body control unit 124) and a second control unit (the lower body control unit 126) are provided separately on the right and left sides of the midline, thereby preventing the first control unit and the second control unit from colliding and interfering with each other. The electrodes on the buttocks of the lower body clothing portion 122 may preferably be provided at positions not in contact with the saddle of the bike.

The bike training is linked to an exercise with an electrical muscle stimulation device 10 by linking the bike and an exercise control device 12 or by installing an exercise control device 12 within the bike. On the display unit 74 of the exercise control device 12, the intensity of the bike exercise and the intensity of the electrical muscle stimulation device 10 may be displayed, and such intensities may be displayed in a ranking format among multiple users. In the ranking, besides the overall ranking, the ranking of the intensity of the bike exercise and the ranking of the intensity of the electrical muscle stimulation device 10 may be displayed separately. Besides the exercise intensity, calories and heart rate may be further displayed on the display unit 74. This not only encourages competition among users but also facilitates guidance by an instructor with the objective information displayed.

Load control using a load adjustment mechanism of the bike may be linked to the control of the intensity of the electrical muscle stimulation device 10. Alternatively, without the load control of the bike, the exercise intensity may be adjusted only with the voltage value of the electrical muscle stimulation device 10. For example, adjustment may be made such that, when the number of revolutions of the bike is smaller than a predetermined number of revolutions in a predetermined time, the voltage value of the electrical muscle stimulation device 10 is increased, and, when the number of revolutions of the bike is equal to or larger than the predetermined number of revolutions in the predetermined time, the voltage value of the electrical muscle stimulation device 10 is decreased. Alternatively, adjustment may be made such that, when the user's heart rate is less than a predetermined percentage of the maximum heart rate, the voltage value of the electrical muscle stimulation device 10 is increased, and, when the user's heart rate is equal to or greater than the predetermined percentage, the voltage value of the electrical muscle stimulation device 10 is decreased.

Also, by providing electrode parts for the soles on the bike pedals and applying a voltage thereto, hybrid training that combines electrical stimulation to the soles and calves with the bike training may be realized.

When so-called "pedaling out of the saddle", which is pedaling while standing on the pedals without sitting on the saddle, is detected, the intensity of electrical stimulation to the upper body (particularly the arms) may be reduced to increase safety. To detect the "pedaling out of the saddle", a contact sensor on a dedicated grip to be held during pedaling out of the saddle, a sensor for reading a change in pedal load, a sitting sensor on the saddle, and the like may be installed.

By providing control of switching, based on the pedaling position, the electrodes to which a voltage is applied, an additional load can be applied to the muscles provided with a load by pedaling. In this case, to detect the pedaling position, the sensor for reading a change in pedal load may be used.

If the bike is equipped with an emergency stop button, control may be provided such that, when the emergency stop button is pressed, the load adjustment mechanism of the bike is set to a high load at once to apply the brakes, and, at the same time, the exercise control device 12 stops the application of voltage by the electrical muscle stimulation device 10. Alternatively, the exercise control device 12 may be equipped with an emergency stop button used to stop the application of voltage by the electrical muscle stimulation device 10. Accordingly, control may be provided such that, when the emergency stop button is pressed, the application of voltage is stopped, and, at the same time, the load adjustment mechanism of the linked bike is set to a high load at once. Alternatively, control may be provided such that, when an abnormality is detected in the user's heart rate, the exercise control device 12 stops the application of voltage by the electrical muscle stimulation device 10, and, at the same time, the load adjustment mechanism of the linked bike is set to a high load at once.

The present invention has been described with reference to an embodiment. The embodiment is intended to be illustrative only, and it will be obvious to those skilled in the art that various modifications to a combination of constituting elements or processes could be developed and that such modifications also fall within the scope of the present invention. Such modifications will be recited below.

In a modification, multiple liquid feeding tubes, which are connected respectively to the multiple electrode parts 30 to feed water thereto, may be provided at appropriate positions in the upper body clothing portion 120 and the lower body clothing portion 122. The multiple liquid feeding tubes are thin flexible tube members made of resin or the like, which each are connected at one end to the conductive polymer fabric 31 of an electrode part 30 and connected at the other end to a predetermined liquid feed pump and a water tank. The multiple liquid feeding tubes are arranged at the respective positions, similarly to the electrical cables 36. This facilitates maintaining the water retention of the electrode parts 30, without the use of sprays or the like.

In a modification, on the floor of the fitness gym 80 where group training as the third exercise program is carried out, electrodes made of conductive paste, such as silver particles, may be printed, and an electrical muscle stimulation device may be implemented by applying a voltage to the electrodes. In this case, the electrodes are printed at predetermined positions on the floor that part of the user's body may come into contact with when the user takes a certain posture in an exercise program. For example, the user may receive electrical stimulation on the soles of the feet while performing an exercise for stretching an Achilles tendon or an exercise for twisting the body with one leg forward and the other back. When a foot comes into contact with one of the electrodes and a current is conducted, an LED around the electrode may be set to light up.

In a modification, the fitness wear 102 may be used in hybrid training that combines voluntary movement produced by consciously moving the body by means of a treadmill or equipment for step-up and down exercise, with involuntary movement produced by the electrical muscle stimulation device 10.

In a modification, the fitness wear 102 may be provided with a means for storing an aromatic or the like. In this case, odors of sweat and the like emitted as a result of training can be alleviated. As the material of the fitness wear 102, a material with low breathability (such as chloroprene rubber or polyurethane) may be employed. In this case, the exercise effect can be improved by perspiration, and the current conductivity can be stabilized. Conversely, as the material of the fitness wear 102, a material with high breathability may be employed. In this case, wash and dry will be easier.

There may be provided a component for connecting the upper body clothing portion 120 and the lower body clothing portion 122 of the fitness wear 102. In this case, shifting of the garments during training can be restrained. The connection positions may be on the left and right sides of the back off the spine. Since the human body has a wider movable range in forward bending, it is preferable to connect the clothing portions at the back. Also, a scale may be provided on the belt or hook-and-loop fastener area. This serves as a guide for adjustment and makes a person other than the wearer perform operation for tightening the fitness wear more easily.

Also, a storage part or an attachment part for a speaker may be provided in the fitness wear 102 so that a small speaker can be attached or fixed to a region around the neck, such as the base of the throat or a shoulder, or a region near an ear or the head of the user. As a means for detachably fixing a speaker to a region around the neck in the fitness wear 102, a snap fastener, a hook, or a hook-and-loop fastener may be used, for example, so that displacement or a fall of the speaker can be prevented. By placing the speaker in a location where audio can be easily heard, even in an environment where multiple users are exercising at the same time, such as a fitness gym, each user can easily hear the sound of his or her own training without being disturbed by sound from neighboring users. In other words, each user can recognize the content of his or her own training, the rhythm of movements, and the correctness of movements, without mistaking them for the movements of other users.

### Second Embodiment

The second embodiment differs from the first embodiment mainly in structure for fixing a control unit 128 to a control unit connection part 20. In the following, description will be given mainly for the differences from the first embodiment, and the explanation of features in common will be omitted as appropriate.

The control unit 128 includes the upper body control unit 124 and the lower body control unit 126, and the upper body control unit 124 is attached to the first control unit connection part 20a, and the lower body control unit 126 is attached to the second control unit connection part 20b. The upper body control unit 124 and the lower body control unit 126 apply electrical stimulation to different regions and provide control differently. Accordingly, it is necessary to prevent a user from accidentally attaching the upper body control unit 124 to the second control unit connection part 20b and the lower body control unit 126 to the first control unit connection part 20a. Therefore, in the attachment structure of each of the upper body control unit 124, the lower body control unit 126, the first control unit connection part 20a, and the second control unit connection part 20b, a structure for preventing a mix-up is provided.

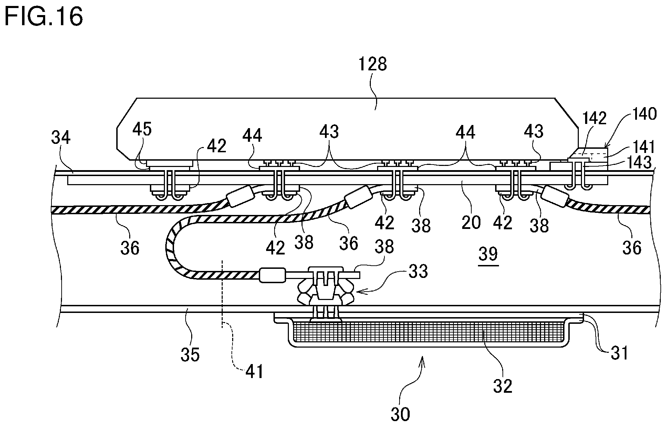

FIG. 16 is a partially enlarged sectional view that illustrates arrangement and structures of a clothing portion, an electrode, electrical cables, a control unit, and a control unit connection part in the second embodiment. To the control unit connection part 20 of planar shape made of resin, a concave clasp 140 is attached with rivets 143 to form a recess 141 of laterally long slit shape at an end of the control unit connection part 20, with the outer fabric 34 sandwiched in between. At an end of the control unit 128, a convex clasp 142 made of resin is formed, and the convex clasp 142 is fitted into the recess 141 of the concave clasp 140, so that one end side of the control unit 128 is fixed to the control unit connection part 20. The other end side of the control unit 128 is also fixed to the control unit connection part 20 by means of a clasp such as the magnetic button 45.

The control unit 128 and the control unit connection part 20 are electrically connected, with the spring connectors 43 being in contact with the pad connectors 44. Since the pad connectors 44, the magnetic button 45, the concave clasp 140, and the like are fixed to the control unit connection part 20 such as to sandwich the outer fabric 34, when the outer fabric 34 is pulled with the movement of the user wearing the fitness wear 102, displacement of the pad connectors 44 may occur. In addition, deforming pressure, such as bending or distortion, may be applied to the control unit connection part 20. In this regard, however, since the connection between the spring connectors 43 and the pad connectors 44 is not fitting connection but contact connection, movements including displacement of pad connectors 44 and deformation of the control unit connection part 20 can be released, thus preventing destruction of the connection structure. In addition, since the connection between the spring connectors 43 and the pad connectors 44 has elasticity, even if pushing force is applied to the control unit 128 from the outside, such pressure can be absorbed. Furthermore, even when inertia or centrifugal force acts, with the movement of the user, such that the control unit 128 moves away from the control unit connection part 20, the extension force of the spring connectors 43 can maintain the electrical connection between the spring connectors 43 and the pad connectors 44.

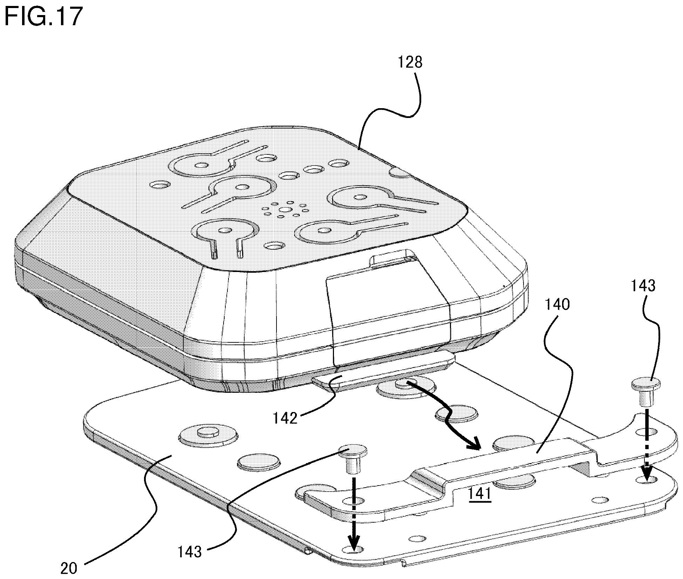

FIG. 17 illustrates attachment structures of a control unit 128 and a control unit connection part 20 in the second embodiment. To an end of the control unit connection part 20, the concave clasp 140 is attached from the above with two rivets 143 such that the outer fabric 34, not illustrated, is sandwiched therebetween. Accordingly, the concave clasp 140 is attached to the control unit connection part 20, and the recess 141 is formed between the control unit connection part 20 and the concave clasp 140. With the convex clasp 142 of the control unit 128 fitted into the recess 141, the control unit 128 is fixed to the control unit connection part 20.

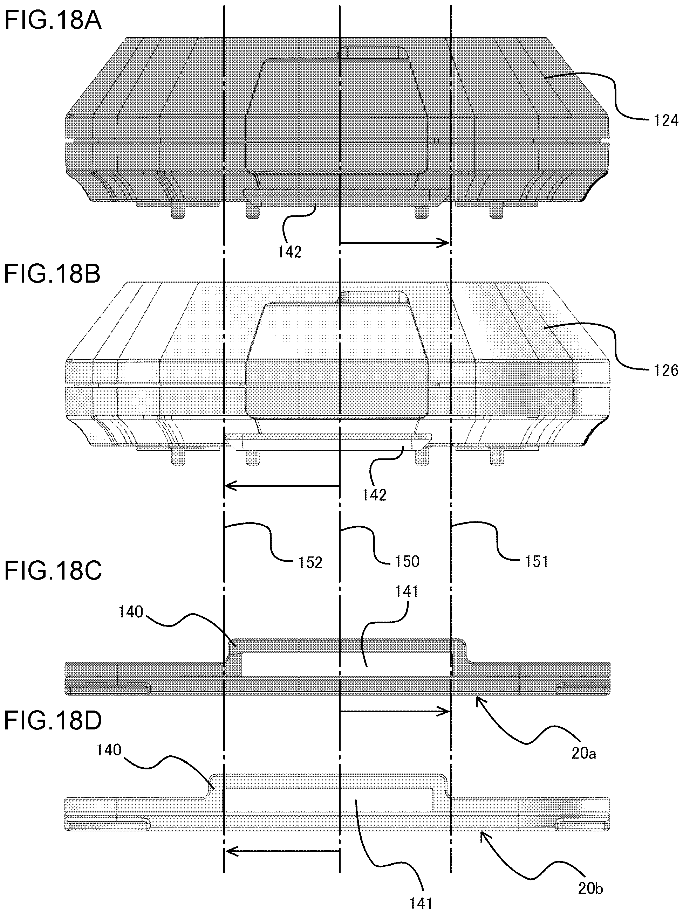

FIGS. 18 illustrate the structures for preventing a mix-up in the second embodiment. FIG. 18A is a front view of the upper body control unit 124, FIG. 18B is a front view of the lower body control unit 126, FIG. 18C is a front view of the first control unit connection part 20a, and FIG. 18D is a front view of the second control unit connection part 20b. Among three dashed dotted lines illustrated in FIGS. 18, a center dashed dotted line 150 is a line indicating the center of each of the upper body control unit 124, lower body control unit 126, first control unit connection part 20a, and second control unit connection part 20b.

The convex clasp 142 of the upper body control unit 124 illustrated in FIG. 18A is provided at a position shifted to the right with respect to the center dashed dotted line 150, and the right end of the convex clasp 142 is in contact with a right dashed dotted line 151 while the left end thereof is not in contact with a left dashed dotted line 152. The recess 141 of the first control unit connection part 20a illustrated in FIG. 18C, to which the upper body control unit 124 is attached, is also provided at a position shifted to the right with respect to the center dashed dotted line 150, and the right end of the recess 141 is in contact with the right dashed dotted line 151 while the left end thereof is not in contact with the left dashed dotted line 152.

Meanwhile, the convex clasp 142 of the lower body control unit 126 illustrated in FIG. 18B is provided at a position shifted to the left with respect to the center dashed dotted line 150, and the left end of the convex clasp 142 is in contact with the left dashed dotted line 152 while the right end thereof is not in contact with the right dashed dotted line 151. The recess 141 of the second control unit connection part 20b illustrated in FIG. 18D, to which the lower body control unit 126 is attached, is also provided at a position shifted to the left with respect to the center dashed dotted line 150, and the left end of the recess 141 is in contact with the left dashed dotted line 152 while the right end thereof is not in contact with the right dashed dotted line 151.

Thus, between the set of the control unit 128 and the control unit connection part 20 for the upper body and the set of the control unit 128 and the control unit connection part 20 for the lower body, there is a difference in installation positions of the convex part and the recess shifted to the left or right side. Accordingly, even if the upper body control unit 124 is to be attached to the second control unit connection part 20b, the upper body control unit 124 cannot be fitted into the second control unit connection part 20b, and, even if the lower body control unit 126 is to be attached to the first control unit connection part 20a, the lower body control unit 126 cannot be fitted into the first control unit connection part 20a. Therefore, a mix-up can be prevented by the structures. The upper body control unit 124 and the first control unit connection part 20a as the set for the upper body are colored with the same color, and the lower body control unit 126 and the second control unit connection part 20b as the set for the lower body are colored with the same color that however is different from the color of the set for the upper body. For example, the set for the upper body may be colored black, and the set for the lower body may be colored gray. In this way, a mix-up is prevented also by making the set for the upper body and the set for the lower body different in color and by coloring each set with the same color.

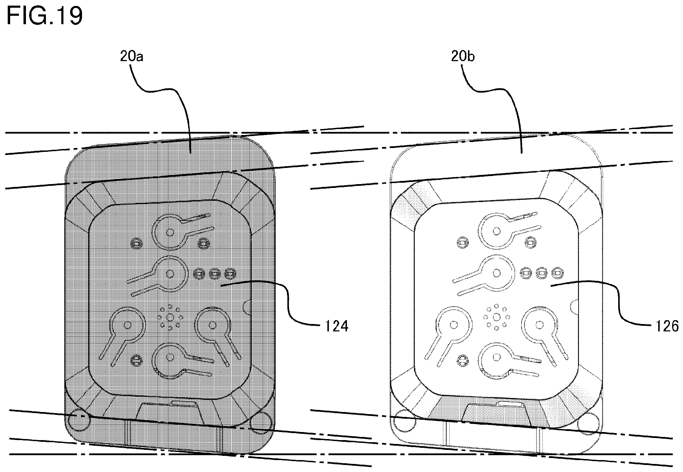

FIG. 19 illustrates structures for preventing an installation mistake in the second embodiment. FIG. 19 is a plan view of the upper body control unit 124 and the first control unit connection part 20a, and the lower body control unit 126 and the second control unit connection part 20b, viewed from the above. As illustrated in FIG. 19, the outer shape of each of the upper body control unit 124, first control unit connection part 20a, lower body control unit 126, and second control unit connection part 20b in plan view is not a regular square but nearly a trapezoidal shape. In each of the outer shapes, the upper side and the lower side are not parallel, and the left side is shorter than the right side when viewed from the above, forming a bilaterally asymmetric shape. Accordingly, if the user remembers the shape with the difference in length of the left and right sides (the difference of the right side being shorter than the left side when viewed from the user at the time of installation) for both the sets for the upper body and the lower body, accidentally attaching a control unit upside down to a control unit connection part 20 can be prevented. In addition, since the upper body control unit 124 and the lower body control unit 126 have the same left side shape and the same right side shape, instead of having horizontally flipped shapes, both the units have the shorter side on the same side. This can prevent confusedly attaching a control unit upside down to a control unit connection part 20. In a modification, the control units 128 and the control unit connection parts 20 may each have a shape other than a trapezoid, such as a half circle and other bilaterally asymmetrical shapes. To prevent the upside-down attachment, the set for the upper body and the set for the lower body have only to be configured in the same nearly symmetrical shape; in addition, to further prevent a mix-up between the set for the upper body and the set for the lower body, a protrusion or a notch may be provided such that the sets differ in only part of the outer shape, for example, so as to be able to distinguish the sets from each other. In this case, such a protrusion or notch may be provided in only one of the set for the upper body and the set for the lower body, or a protrusion may be provided in one of the sets while a notch is provided in the other. Alternatively, the position of a protrusion or a notch may be made different between the set for the upper body and the set for the lower body, so that the sets can be distinguished from each other.

Since the set for the upper body and the set for the lower body have a basic outer shape in common, common parts can be used for the body part of each of the upper body control unit 124 and the lower body control unit 126, except for the base portion including the convex clasp 142. Similarly, common parts can be used for each of the first control unit connection part 20a and the second control unit connection part 20b except for the concave clasp 140. Such commonality of parts can prevent an increase in cost.

FIG. 19 illustrates an example in which the entirety of the upper body control unit 124 and the first control unit connection part 20a are colored black, and the entirety of the lower body control unit 126 and the second control unit connection part 20b are colored gray. In a modification, only the base portion of each of the upper body control unit 124 and the lower body control unit 126 may be colored black or gray, for example. Also, only a portion including the concave clasp 140 of each of the first control unit connection part 20a and the second control unit connection part 20b may be colored black or gray, for example.

### Illustrative Configurations and Arrangement of Electrodes and Control Unit

In the following, illustrative configurations and arrangement of electrodes and a control unit described in the aforementioned first embodiment will be described. In the upper body clothing portion 120 illustrated in FIGS. 5, five pairs of cathodes and anodes are arranged. Considering applying a current between each of the pairs of electrodes, the voltage value to be applied is set for each electrode pair, so that the intensity of electric stimulation is individually set. If all the multiple electrode pairs are designed to be connected to a common ground, a closed circuit may be formed through the human body between electrodes other than those to which a voltage is intended to be applied. Accordingly, if a large potential difference occurs between one electrode pair and another, a leakage current may flow through an unintended internal path. Therefore, a configuration in which separate grounds are provided is employed so that a closed circuit is not formed between electrodes other than those assumed as a pair, especially between electrodes where generation of a leakage current should be prevented. Accordingly, one of the following four illustrative unit configurations related to the electrode configuration and the arrangement of the electrodes is employed. It is assumed here that a group of electrodes around the torso (the first electrode part 30a, second electrode part 30b, third electrode part 30c, fourth electrode part 30d, fifth electrode part 30e, and sixth electrode part 30f) will be referred to as a "first electrode group", and a group of electrodes around the arms (the seventh electrode part 30g, eighth electrode part 30h, ninth electrode part 30i, and the tenth electrode part 30j) will be referred to as a "second electrode group", so as to distinguish the groups from each other. The configuration is then designed to avoid generation of a leakage current between the first electrode group and the second electrode group. As the structure of a control unit, the structure in the first embodiment as illustrated in FIG. 8 may be employed, or the structure in the second embodiment as illustrated in FIGS. 16-19 may also be employed.

### (1) First example

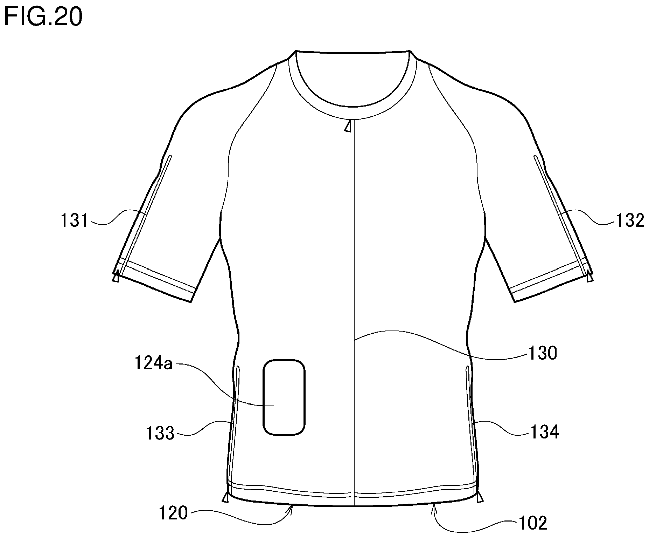

FIG. 20 schematically illustrates appearance of the upper body clothing portion and the upper body control unit in the first example. In the first example, the first electrode group and the second electrode group are controlled by the same upper body control unit (a first upper body control unit 124a), but, as the reference voltages for the respective groups, separate grounds are provided within the same first upper body control unit 124a so that the groups are internally isolated from each other. However, when such an integrated control unit is thus configured by providing separate grounds within a single first upper body control unit 124a for mutual isolation, the size of the first upper body control unit 124a could be about twice the size of the upper body control unit 124 illustrated in FIG. 4 or a control unit in other illustrative configurations described later.

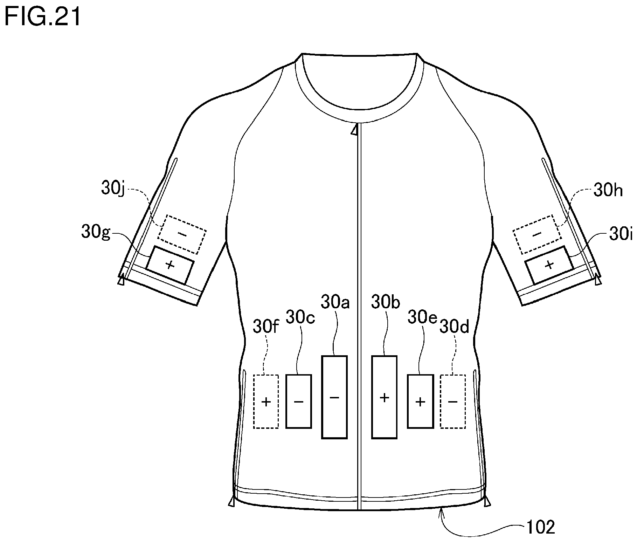

FIG. 21 schematically illustrates arrangement of electrodes in the upper body clothing portion of the first example. In FIG. 21, the electrodes indicated by solid lines are electrodes arranged on the inner fabric on the front side of the upper body clothing portion 120, and the electrodes indicated by dotted lines are electrodes arranged on the inner fabric on the back side of the upper body clothing portion 120. Around the torso, the first electrode part 30a, second electrode part 30b, third electrode part 30c, and fifth electrode part 30e arranged on the front side and the fourth electrode part 30d and sixth electrode part 30f arranged on the back side constitute a first electrode group 330. Also, around the arms, the seventh electrode part 30g and ninth electrode part 30i arranged on the front side and the eighth electrode part 30h and tenth electrode part 30j arranged on the back side constitute a second electrode group 332.

Around the torso, the rectus abdominis muscles and the oblique abdominal muscles are particularly approximate, and, to prevent the formation of a current conduction path between neighboring electrodes that are not intended as a pair, the neighboring electrodes that are not intended as a pair are arranged to have the same polarity. First, one of the first electrode part 30a and the second electrode part 30b arranged on the rectus abdominis muscles is provided as an anode, and the other is provided as a cathode. For example, the first electrode part 30a may be the cathode, and the second electrode part 30b may be the anode. The third electrode part 30c adjacent to the first electrode part 30a as a cathode is then also provided as a cathode to be the same as the first electrode part 30a, and the sixth electrode part 30f paired with the third electrode part 30c is provided as an anode accordingly. Also, the fifth electrode part 30e adjacent to the second electrode part 30b as an anode is also provided as an anode to be the same as the second electrode part 30b, and the fourth electrode part 30d paired with the fifth electrode part 30e is provided as a cathode.

With regard to the size of each electrode, the first electrode part 30a and the second electrode part 30b for the rectus abdominis muscles may be made larger in length and width than the other electrodes. Also, the first electrode part 30a and the second electrode part 30b for men and those for women may be configured to be different in size such that those for men are larger than those for women. Also, since the degree of prominence of the rectus abdominis muscles is generally larger than that of the oblique abdominal muscles, the degree of contact with muscles of the electrodes for the oblique abdominal muscles is considered to be smaller than that of the electrodes for the rectus abdominis muscles. Accordingly, the pad members of the third electrode part 30c, fourth electrode part 30d, fifth electrode part 30e, and sixth electrode part 30f for the oblique abdominal muscles are made thicker than the pad members of the first electrode part 30a and second electrode part 30b for the rectus abdominis muscles. For example, the thickness of the pad member of each electrode for the oblique abdominal muscles may be about twice the thickness of the pad member of each electrode for the rectus abdominis muscles or may be the thickness of two stacked pad members of the electrodes for the rectus abdominis muscles. Similarly, the pad members of the seventh electrode part 30g, eighth electrode part 30h, ninth electrode part 30i, and the tenth electrode part 30j for the arms are made thicker than the pad members of the first electrode part 30a and second electrode part 30b for the rectus abdominis muscles. For example, the thickness of the pad member of each electrode for the arms may be about twice the thickness of the pad member of each electrode for the rectus abdominis muscles or may be the thickness of two stacked pad members of the electrodes for the rectus abdominis muscles. Also, the thickness of the pad member of each of the 19th electrode part 30s, 20th electrode part 30t, 21st electrode part 30u, and 22nd electrode part 30v as the electrodes for the buttocks illustrated in FIGS. 6 may be about twice the thickness of the pad member of another electrode, such as the 11th electrode part 30k, 12th electrode part 301, 13th electrode part 30m, 14th electrode part 30n, 15th electrode part 30o, 16th electrode part 30p, 17th electrode part 30q, or 18th electrode part 30r, or may be the thickness of two stacked pad members of the another electrode.

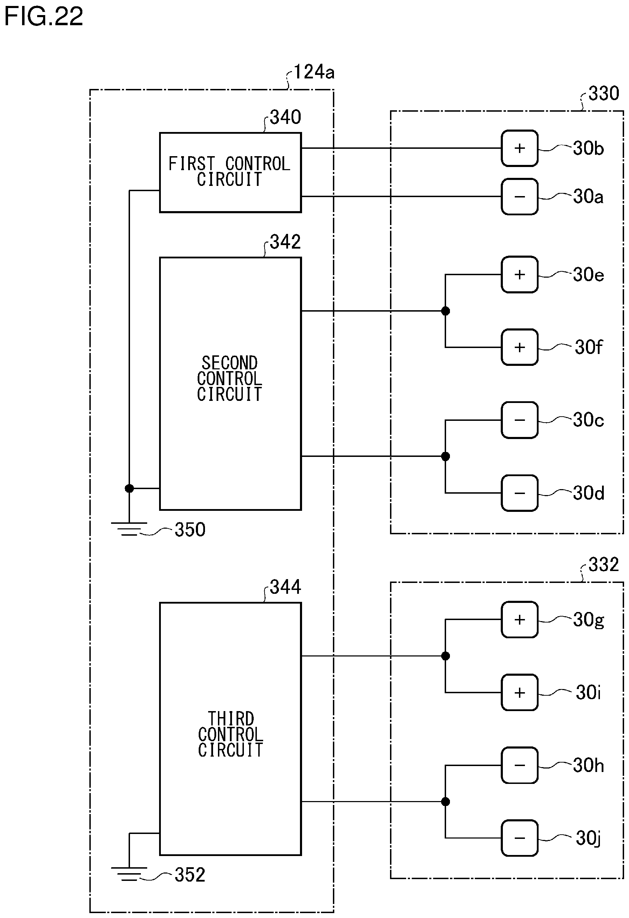

FIG. 22 schematically illustrates an illustrative configuration of the control unit and electrodes in the first example. The first upper body control unit 124a is integrally configured to include a first control circuit 340 that controls a voltage applied to the first electrode part 30a and second electrode part 30b arranged on the rectus abdominis muscles, a second control circuit 342 that controls a voltage applied to the third electrode part 30c, fourth electrode part 30d, fifth electrode part 30e, and sixth electrode part 30f arranged on the oblique abdominal muscles, a third control circuit 344 that controls a voltage applied to the seventh electrode part 30g, eighth electrode part 30h, ninth electrode part 30i, and tenth electrode part 30j arranged on the arms, a first ground 350, and a second ground 352. The first electrode part 30a and second electrode part 30b arranged on the rectus abdominis muscles and the third electrode part 30c, fourth electrode part 30d, fifth electrode part 30e, and sixth electrode part 30f arranged on the oblique abdominal muscles are included in the first electrode group 330. The seventh electrode part 30g, eighth electrode part 30h, ninth electrode part 30i, and tenth electrode part 30j arranged on the arms are included in the second electrode group 332. The first control circuit 340 and the second control circuit 342 that control a voltage applied to each of the electrodes included in the first electrode group 330 are connected to the first ground 350. The third control circuit 344 that controls a voltage applied to each of the electrodes included in the second electrode group 332 is connected to the second ground 352. The system including the first control circuit 340, the second control circuit 342, and the first ground 350 and the system including the third control circuit 344 and the second ground 352 are separately arranged within a casing of the first upper body control unit 124a and electrically isolated from each other. Thus, since the first electrode group 330 and the second electrode group 332 are connected respectively to separate grounds, instead of being connected to a ground in common, generation of a leakage current due to the formation of a closed circuit between unintended electrodes, i.e., between an electrode included in the first electrode group 330 and an electrode included in the second electrode group 332, can be prevented.

### (2) Second example

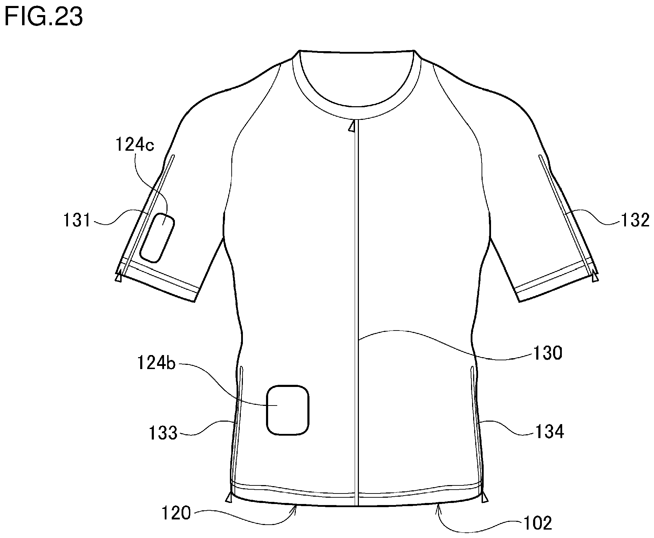

FIG. 23 schematically illustrates appearance of the upper body clothing portion and the upper body control unit in the second example. In the second example, the control unit is divided for the first electrode group and the second electrode group, and a ground as a reference voltage is provided within each control unit. More specifically, the control unit for controlling a voltage applied to each electrode is divided into a second upper body control unit 124b for controlling the first electrode group and a third upper body control unit 124c for controlling the second electrode group. The second upper body control unit 124b is provided on the right flank, similarly to the upper body control unit 124 in FIG. 4 and the first upper body control unit 124a in FIG. 20, and the third upper body control unit 124c is provided on the front side of the right arm. The third upper body control unit 124c may suitably be of a shape and size that does not interfere when worn around the arm. For example, the third upper body control unit 124c may be configured smaller than the second upper body control unit 124b. The second upper body control unit 124b is a control unit of the same size as the upper body control unit 124 illustrated in FIG. 4. The configuration and arrangement of the electrodes are the same as those in the first example shown in FIG. 21.

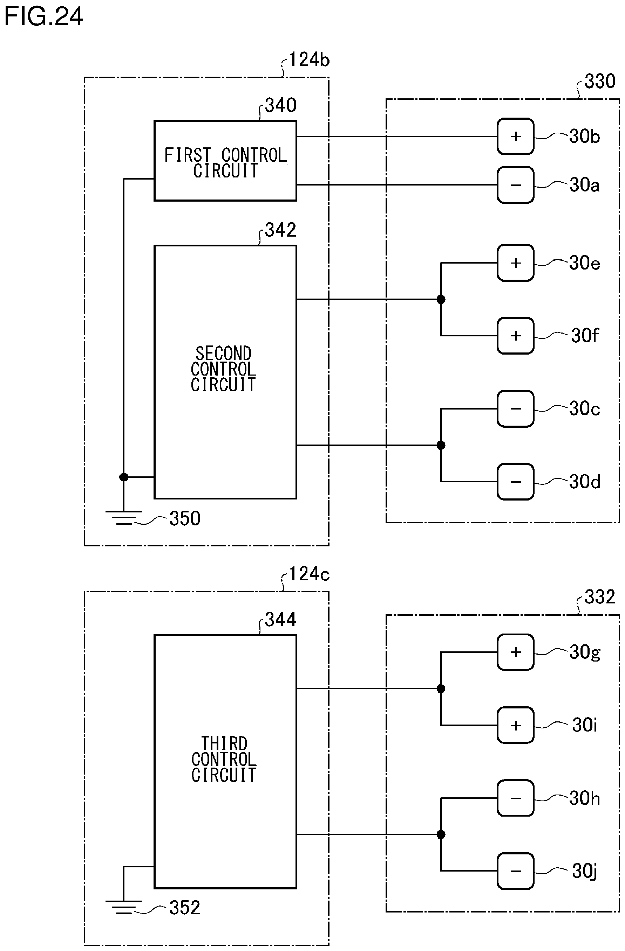

FIG. 24 schematically illustrates an illustrative configuration of the control units and electrodes in the second example. The second upper body control unit 124b includes the first control circuit 340 that controls a voltage applied to the first electrode part 30a and second electrode part 30b arranged on the rectus abdominis muscles, the second control circuit 342 that controls a voltage applied to the third electrode part 30c, fourth electrode part 30d, fifth electrode part 30e, and sixth electrode part 30f arranged on the oblique abdominal muscles, and the first ground 350. The third upper body control unit 124c includes the third control circuit 344 that controls a voltage applied to the seventh electrode part 30g, eighth electrode part 30h, ninth electrode part 30i, and tenth electrode part 30j arranged on the arms, and the second ground 352. The first electrode part 30a and second electrode part 30b arranged on the rectus abdominis muscles and the third electrode part 30c, fourth electrode part 30d, fifth electrode part 30e, and sixth electrode part 30f arranged on the oblique abdominal muscles are included in the first electrode group 330. The seventh electrode part 30g, eighth electrode part 30h, ninth electrode part 30i, and tenth electrode part 30j arranged on the arms are included in the second electrode group 332. The first control circuit 340 and the second control circuit 342 that control a voltage applied to each of the electrodes included in the first electrode group 330 are connected to the first ground 350. The third control circuit 344 that controls a voltage applied to each of the electrodes included in the second electrode group 332 is connected to the second ground 352. The system including the first control circuit 340, the second control circuit 342, and the first ground 350 and the system including the third control circuit 344 and the second ground 352 are arranged respectively within the divided separate control units and electrically isolated from each other. Thus, since the first electrode group 330 and the second electrode group 332 are connected respectively to separate grounds, instead of being connected to a ground in common, generation of a leakage current due to the formation of a closed circuit between unintended electrodes, i.e., between an electrode included in the first electrode group 330 and an electrode included in the second electrode group 332, can be prevented.

### (3) Third example

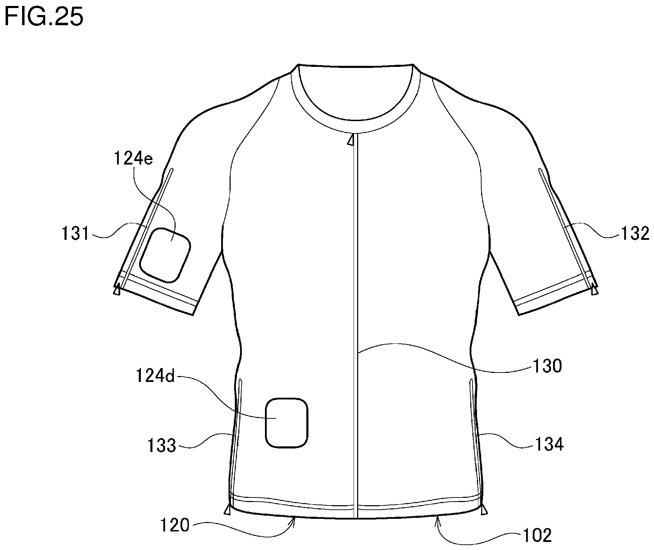

FIG. 25 schematically illustrates appearance of the upper body clothing portion and the upper body control unit in the third example. As is the case in the second example, also in the third example, the control unit is divided for the first electrode group and the second electrode group, and a ground as a reference voltage is provided within each control unit. More specifically, the control unit for controlling a voltage applied to each electrode is divided into a fourth upper body control unit 124d for controlling the first electrode group and a fifth upper body control unit 124e for controlling the second electrode group. The fourth upper body control unit 124d is provided on the right flank, similarly to the second upper body control unit 124b in FIG. 23, and the fifth upper body control unit 124e is provided on the front side of the right arm, similarly to the third upper body control unit 124c in FIG. 23. However, a unit with parts in common is used for each of the fourth upper body control unit 124d and the fifth upper body control unit 124e to reduce the manufacturing cost. The configuration and arrangement of the electrodes in the third example are the same as those in the first example shown in FIG. 21.

An illustrative configuration of the control units and electrodes in the third example is the same as that in the second example shown in FIG. 24. More specifically, the fourth upper body control unit 124d includes the first control circuit 340 that controls a voltage applied to the first electrode part 30a and second electrode part 30b arranged on the rectus abdominis muscles, the second control circuit 342 that controls a voltage applied to the third electrode part 30c, fourth electrode part 30d, fifth electrode part 30e, and sixth electrode part 30f arranged on the oblique abdominal muscles, and the first ground 350. The fifth upper body control unit 124e includes the third control circuit 344 that controls a voltage applied to the seventh electrode part 30g, eighth electrode part 30h, ninth electrode part 30i, and tenth electrode part 30j arranged on the arms, and the second ground 352. The system including the first control circuit 340, the second control circuit 342, and the first ground 350 and the system including the third control circuit 344 and the second ground 352 are arranged respectively within the divided separate control units and electrically isolated from each other. Thus, also in the third example, since the first electrode group 330 and the second electrode group 332 are connected respectively to separate grounds, instead of being connected to a ground in common, generation of a leakage current due to the formation of a closed circuit between unintended electrodes, i.e., between an electrode included in the first electrode group 330 and an electrode included in the second electrode group 332, can be prevented.

### (4) Fourth example

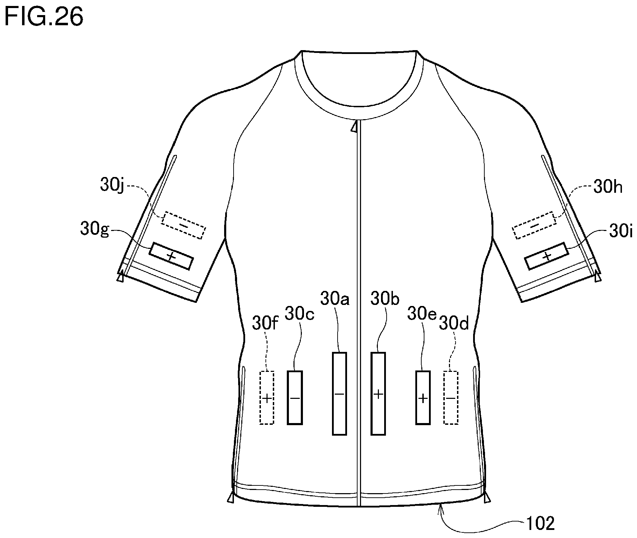

FIG. 26 schematically illustrates size and arrangement of electrodes in the upper body clothing portion of the fourth example. The function to allow a user to set an arbitrary intensity as the intensity of electrical stimulation for each electrode pair is omitted, and the voltage is controlled within a limited range in which a potential difference that may cause a leakage current between unintended electrodes is not produced. When the voltage applied between the electrodes and the current flowing between the electrodes are constant, the greater the distance between the paired electrodes or the greater the size of the paired electrodes, the greater the sensory intensity of the electrical stimulation, until a predetermined critical value is reached. Using such characteristics, in the fourth example, the distance and size of the electrodes is adjusted such that the sensory intensity becomes larger than in the other illustrative configurations, instead of making the voltage value applied between at least some of the electrodes smaller than in the other illustrative configurations, thereby maintaining the sensory intensity while reducing the applied voltage. Each of the electrodes in FIG. 26 is disposed at a position similar to that of each of the electrodes in the first example shown in FIG. 21. However, each electrode in FIG. 26 has a narrower width and a smaller area than each electrode in FIG. 21, so that the sensory intensity is adjusted to be relatively smaller; meanwhile, the distance between the paired electrodes in FIG. 26 is larger than the distance between the paired electrodes in FIG. 21, so that the sensory intensity is adjusted to be relatively larger. Also, the maximum value of the applied voltage is limited to a value smaller than the maximum value in the first example. FIG. 26 shows an example in which the distance and size of all the paired electrodes is adjusted; as a modification, some of the paired electrodes may be configured to have the same distance and size as the corresponding electrodes shown in FIG. 21. In the fourth example, the control unit is not divided for the first electrode group and the second electrode group but is configured as an integral control unit, as shown in FIG. 4. Also, grounds are not separately provided, and each electrode is connected to a single ground. However, in a modification, grounds may be separately connected within the control unit as in the first example, or control units may be separately configured as in the second and third examples.

In this way, with the limitation of the voltage range between electrodes and the adjustment of the distance between electrodes and the size of the electrodes, a balance is achieved such that the sensory intensity is not reduced. This provides a configuration in which a potential difference that may cause a leakage current between unintended electrodes is not produced. The optimal combination of the range of applied voltage between electrodes, the distance between electrodes, and the size of the electrodes may be obtained through experiments using various combinations with each element replaced.

In the first through fourth examples, the timing for applying a current to the first electrode group 330 and the timing for applying a current to the second electrode group 332 may be made different so that each of the electrode groups operates exclusively, and the overlap of the timings for applying a current may be avoided. Accordingly, a large potential difference that may form a path of a leakage current between unintended electrodes may be prevented from occurring.

As a modification, the configuration and arrangement of electrodes as illustrated in FIGS. 15 may be employed. More specifically, electrodes may be configured and arranged such that two electrodes are vertically arranged on each of the left and right rectus abdominis muscles to apply electrical stimulation in a vertical direction. The electrodes for the rectus abdominis muscles in this case are included in the first electrode group 330 as in the first through fourth examples. Further, electrodes may be configured and arranged such that two electrodes are horizontally arranged also on each of the left and right pectoralis major muscles to apply electrical stimulation in a horizontal direction. The electrodes for the pectoralis major muscles in this case may be configured to be included in the second electrode group 332 as in the first through fourth examples. Alternatively, the electrodes for the pectoralis major muscles may be configured to be included in a different electrode group other than the first electrode group 330 and the second electrode group 332, and the different electrode group may be connected to a different ground to which the first electrode group 330 or the second electrode group 332 is not connected. Also in this case, generation of a leakage current between the first electrode group 330 and the second electrode group 332 can be avoided.

### INDUSTRIAL APPLICABILITY

The present invention relates to clothing for exercise.

### REFERENCE SIGNS LIST

- 10: electrical muscle stimulation device
- 28: control section
- 30: electrode part
- 31: conductive polymer fabric
- 32: pad member
- 36: electrical cable
- 39: closed space
- 70: control section
- 102: fitness wear

## Claims

1. Electrical stimulation fitness wear, comprising:
a clothing portion wearable on a body;
a plurality of electrode parts provided in the clothing portion, at positions that respectively face a plurality of target body parts such as to be respectively in proximity to the plurality of target body parts, so as to provide electrical stimulation to the plurality of target body parts, the plurality of electrode parts each having a surface formed of a conductive polymer fabric;
a control unit that controls a voltage of the plurality of electrode parts;
a connection part electrically connected to the control unit; and
a plurality of electric wires that respectively connect the plurality of electrode parts to the connection part electrically, wherein
the entirety or part of the clothing portion has a multi-layered structure in which a plurality of fabrics overlap each other and also includes, between the plurality of fabrics, a closed space formed by sewing the entirety or part of the circumference thereof,
the plurality of electric wires include a first electric wire and a second electric wire and are arranged in the closed space, and
the plurality of electrode parts include a first electrode part to which the first electric wire is connected and a second electrode part to which the second electric wire is connected, and the first electrode part and the second electrode part are provided at separate positions such that a current can be applied to a predetermined body part between the electrodes.

2. Electrical stimulation fitness wear, comprising:
a clothing portion that is stretchable and wearable on a body;
a plurality of electrode parts provided at positions that respectively face a plurality of target body parts such as to be respectively in proximity to the plurality of target body parts, so as to provide electrical stimulation to the plurality of target body parts;
a connection part electrically connected to a control unit that controls a voltage of the plurality of electrode parts; and
a plurality of electric wires that are stretchable and respectively connect the plurality of electrode parts to the connection part electrically.

3. The electrical stimulation fitness wear according to claim 2, wherein
at least part of the clothing portion has a multi-layered structure in which a plurality of fabrics overlap each other and also includes, between the plurality of fabrics, a closed space formed by sewing at least part of the circumference thereof, and
the electric wires each have stretchability such as to stretch and contract in the wake of stretching and contraction of the clothing portion, and part of the each electric wire is fixed to a predetermined fixing part of at least one of the plurality of fabrics.

4. The electrical stimulation fitness wear according to any one of claims 1 through 3, wherein
the electrode parts each include a pad member provided on the clothing portion, on the side facing the skin of the wearer, and also include a conductive polymer fabric that covers the pad member, and
the electric wires are each connected to a corresponding conductive polymer fabric via a connector that penetrates a fabric of the clothing portion to electrically connect the outer side and inner side thereof.

5. The electrical stimulation fitness wear according to any one of claims 1 through 4, wherein
the clothing portion includes an upper body garment and a lower body garment, in each of which the plurality of electrode parts and the plurality of electric wires are provided,
to the clothing portion, a first control unit and a second control unit are attached as the control unit,
the first control unit is attached to the front of the upper body garment, on a first side as one of the left and right sides of the midline, and
the second control unit is attached to the front of the lower body garment, on a second side that is different from the first side of the left and right sides of the midline.
